# EUROPEAN PATENT APPLICATION

(11) **EP 4 201 440 A1**
(43) Date of publication of application: **28.06.2023**
(21) Application number: 21217755.4
(22) Date of filing: 24.12.2021
(51) Int. Cl.: A61L 31/04, A61L 31/14, A61L 15/00

(54) **IMPLANTABLE DEVICE COMPRISING A HYDROGEL**

(71) Applicant: Adocia, 69003 Lyon (FR)
(72) Inventor: GEISSLER, Alexandre, 69330 Meyzieu (FR)
(74) Representative: Tripoz, Inès

(57) **Abstract**

The invention concerns an implantable device comprising at least a hydrogel which may incorporate:
- active principles, such as peptides, hormones or proteins, or
- secreting cells, which may be cells secreting peptides or hormones.

The aim of this device is to prevent, treat or cure disease. In particular, this could allow the prevention and/or treatment of chronic diseases by replacing totally or in part the function of natural occurring cells which are deficient in the patients. The invention also concerns the crosslinked polymer, which is a part of the hydrogel or which constitutes the hydrogel, and its precursors.

## Description

The domain of the invention is cell therapy. More particularly the invention is about an implantable device comprising at least a hydrogel which may incorporate:
- active principles, such as peptides, hormones or proteins, or
- secreting cells, which may be cells secreting peptides or hormones.

The aim of this device is to prevent, treat or cure disease. In particular, this could allow the prevention and/or treatment of chronic diseases by replacing totally or in part the function of natural occurring cells which are deficient in the patients. The invention also concerns the crosslinked polymer, which is a part of the hydrogel or which constitutes the hydrogel, and its precursors.

The cells may be isolated or aggregated and may be chosen from group of cells that pertains to one type or of different types.

Hydrogels can be used in multiple systems like:
- implantable device for controlled drug or active pharmaceutical ingredient release or
- implantable device comprising cells.

Hydrogels comprise or consist of polymers that are cross-linked in a 3D network. They can either be natural or synthetic, homopolymers or copolymers. They have the ability to absorb and retain large amounts of water. This is known as the swelling of hydrogels.

The said network may be constituted by grafting and/or chemical crosslinking by creating bonds between the polymeric chains, these bonds possibly being covalent bonds. The said network may also be obtained by transient physical interactions, for example ionic, hydrophobic or hydrogen bonds. Hydrogels have the ability to absorb and retain large amounts of water. This is known as the swelling of hydrogels and may be due to the hydrophilic nature of some of the functional groups (like alcohols, carboxylates, etc.) of the backbone chain.

In order to have a device which may be implantable and able to host cells and deliver active principles on the long run, many features have to be obtained.

Among these features may be cited:
- a low degradation rate, in particular a low biodegradability rate, or a good in vivo stability, in order for the hydrogel to keep its integrity and for cells not to go in the organism of the patient,
- an appropriate permselectivity, allowing a low, or even better no, immune response by isolating the incorporated cells, totally or in part, from the immune system of the host, while allowing the passage of the active principle, for example hormone, peptide or protein,
- a good mitigation of the foreign body response, or a good biocompatibility, in particular a low cytotoxicity and a good local tolerance,
- allowing the cells to have a high survival rate, such has a vascularisation close enough to the cells in order to deliver oxygen and nutriments,
- allowing the cells to have a good functionality within the hydrogel,
- an appropriate homogeneity, for example the hydrogel being transparent or translucid is a good sign of it.

Among particularly interesting feature for such an implantable device may be cited
- a good integration of the device in the body of the patient, in particular
- no, or almost none, rejection and fibrotic response leading to a partial or total "encapsulation" of the device by a body response,
- development of vascularisation close to the device, for example to lead to a good survival rate and to a good functional level of the cells.

In order to be used as controlled release systems or scaffolds for cells, hydrogels must have particular characteristics so as to exhibit all or part of the desired properties such as disclosed above as well as good mechanical and rheological properties.

Among the rheological and mechanical properties that are of high interest for the hydrogel may be cited:
- a good homogeneity, which can be linked to a good transparency or translucency,
- appropriate resistance and flexibility toward stress and strain mechanics, in particular when being handled and implanted,
- a defined mesh size to maximise oxygen and nutrients exchanges, controlled transport properties and permselectivity,
- a good stability in vivo, i.e. resistance to enzymatic or oxidative degradation,
- a good integration in the tissue and in particular a good vascularization of or around the device.

Among parameters which can give indications on the rheological and mechanical desired properties may be cited:
- tan δ (called loss tangent) which gives indication on mechanical properties,
- G', which gives indication on the mesh size and on the elastic properties,
- compression and/or traction deformation at break, which gives indication on the elasticity and resistance of the hydrogel,
- swellability, which gives indications on mechanical properties.

The suitability of hydrogel depends on its bulk structure. An important parameter used to characterize the network structure of hydrogel according to the invention are the polymer volume fraction in the swollen state, the molecular weight of the polymer chain between two neighbouring crosslinking points, and the corresponding mesh size.

In order to be used as controlled release systems or scaffolds for cells, hydrogels must have particular characteristics so as to exhibit all or part of the desired properties such as disclosed above.

The underlying problem is solved totally or in part by the provision of an hydrogel that presents physicochemical properties allowing the manufacture of an implantable device, biocompatibility properties and a design that allow cells survival.

Moreover, and on the contrary to many prior arts, the hydrogels according to the invention have tunable features, considering the precursors used and the way the crosslinking is performed. This can lead to hydrogels having a controlled incorporation and release of specific objects from the hydrogel.

One of the main problems solved by the invention concerns the cells survival and/or the improvement of exchanges with the outside of the hydrogel.

The problems are solved totally or in part by the provision of an implantable device comprising at least a hydrogel, said device having a geometric shape, such as a parallelepiped or a disc.

The invention concerns an implantable device comprising at least a hydrogel comprising:
- an upper face,
- a bottom face parallel to the upper face, the distance between the upper and the bottom faces defining a thickness of the device
- at least one lateral face between the upper and bottom faces, and
- an array of channels within the hydrogel, each channel extending from the bottom face and/or from the upper face,
   characterized in that
- the distance between two neighboring channels of the array of channels, according to a dimension orthogonal to the thickness of the hydrogel, is inferior or equal to 1000 µm.

The "distance between channels" being the distance between the closest edges of the considered channels.

The invention also concerns an implantable device comprising at least a hydrogel comprising:
- an upper face,
- a bottom face parallel to the upper face, the distance between the upper and the bottom faces defining a thickness of the device
- at least one lateral face between the upper and bottom faces, and
- an array of channels within the hydrogel, each channel extending from the bottom face and/or from the upper face,
   characterized in that
- at least 90 % of the channels have at least 2, in particular at least 3, and more particularly at least 4, channels at a distance, according to a dimension orthogonal to the thickness of the hydrogel, inferior or equal to 1000 µm.

Within the scope of the present invention
- by "neighbouring channels" is meant two channels of a group of adjacent channels between which the distance is a minimum. and
- by "adjacent channels" is meant two channels positioned side by side and not separated by another channel.

In an embodiment, at least 50 % of the surface of the hydrogel is directly in contact with the medium in which the device is implanted.

In an embodiment, at least 75 % of the surface of the hydrogel is directly in contact with the medium in which the device is implanted.

In an embodiment, at least 90 % of the surface of the implantable device comprising at least a hydrogelis directly in contact with the medium in which the device is implanted.
In an embodiment, at least 95 % of the surface of the implantable device comprising at least a hydrogel is directly in contact with the medium in which the device is implanted.

In an embodiment, 99 % of the surface of the implantable device comprising at least a hydrogel is directly in contact with the medium in which the device is implanted.

In an embodiment, at least 50 % of the surface of the hydrogel is directly in contact with the exterior of the device.

By « directly in contact with the exterior » means there is no separation between the hydrogel and the exterior, for example no wall made of a non-hydrogel material between the hydrogel and the exterior of the device.

In an embodiment, at least 75 % of the surface of the hydrogel is directly in contact with the exterior.

In an embodiment, at least 90 % of the surface of the hydrogel is directly in contact with the exterior.

In an embodiment, at least 95 % of the surface of the hydrogel is directly in contact with the exterior.

In an embodiment, at least 99 % of the surface of the hydrogel is directly in contact with the exterior.

In an embodiment, 100 % of the surface of the hydrogel is directly in contact with the exterior.

According to an embodiment, the hydrogel comprises a first plurality of polymers which are crosslinked via a crosslinker, the crosslinking being obtained via covalent bonding.

Different embodiments of the device according to the invention will be described with reference to the figures.

In these different figures, equivalent elements are designated by the same numerical reference.

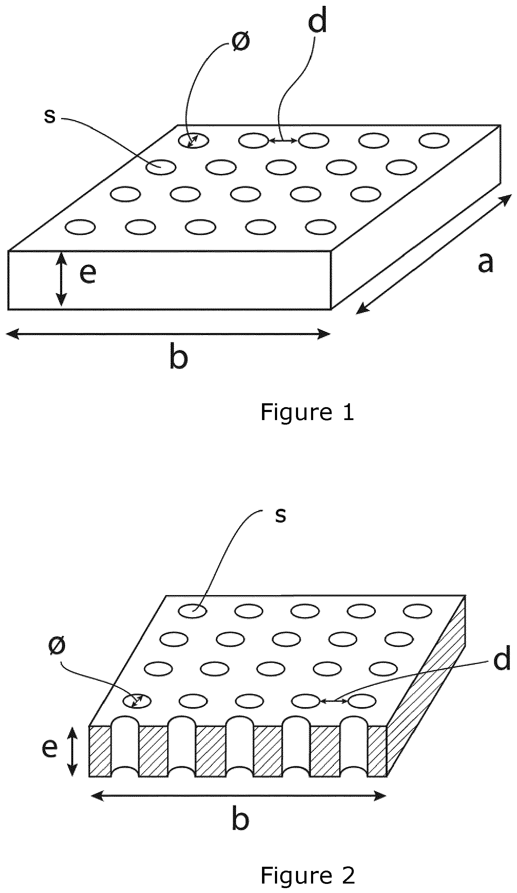

Referring to Figure 1, the device comprises:
- a parallepipedic shaped hydrogel of thickness e, length b and width a, comprising circular through holes or lumen p that have a diameter ∅, the distance between two neighbouring holes or lumen being d,

Figure 2 is a cross sectional view of the parallepipedic shaped hydrogel where one can see that the circular holes or lumen p are through,

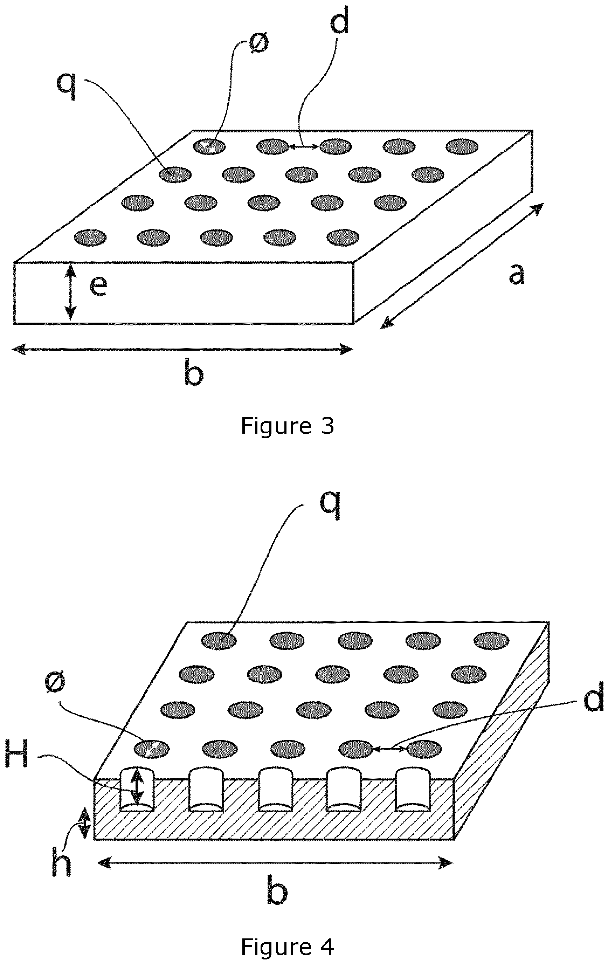

Referring to Figure 3, the device comprises:
- a parallepipedic shaped hydrogel of thickness e, length b and width a, comprising circular blind holes or lumen q on the upper face that have a diameter ∅, the distance between two neighbouring holes or lumen being d,

Figure 4 is a cross sectional view of the parallepipedic shaped hydrogel where one can see that the circular holes or lumen q are blind and that their depth is H, and the distance between the bottom of the holes and the bottom surface of the parallepipedic shaped hydrogel is h.

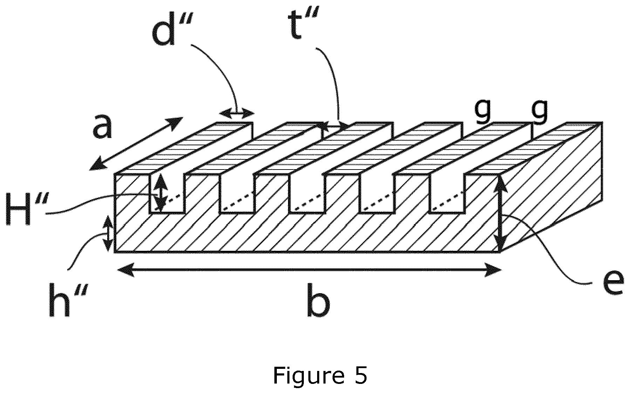

Figure 5 is a a cross-sectional view of a device comprising:
- a parallepipedic shaped hydrogel of thickness e, length b and width a, comprising grooves g on the upper face whose width is t", the distance between two grooves g being d",the depth of the grooves bein H" and the distance between the bottom of the grooves and the bottom surface of the parallepipedic shaped hydrogel is h".

According to an embodiment the thickness e of the implantable device comprising at least a hydrogel is less than or equal to 5 mm.

According to an embodiment the thickness e of the implantable device comprising at least a hydrogel is less than or equal to 3 mm.

According to an embodiment the thickness e of the implantable device comprising at least a hydrogel is less than or equal to 2 mm.

According to an embodiment the thickness e of the implantable device comprising at least a hydrogel is less than or equal to 1 mm.

According to an embodiment the thickness e of the implantable device comprising at least a hydrogel is less than or equal to 500 µm.

According to an embodiment the thickness e of the implantable device comprising at least a hydrogel is more than or equal to 200 µm.

According to an embodiment the thickness e of the implantable device comprising at least a hydrogel is more than or equal to 250 µm.

According to an embodiment the thickness e of the implantable device comprising at least a hydrogel is more than or equal to 300 µm.

According to an embodiment the thickness e of the implantable device comprising at least a hydrogel is more than or equal to 350 µm.

According to an embodiment the thickness e of the implantable device comprising at least a hydrogel is more than or equal to 400 µm.

According to an embodiment the thickness e of the implantable device comprising at least a hydrogel is comprised between 200 and 5000 µm.

According to an embodiment the thickness e of the implantable device comprising at least a hydrogel is comprised between 250 and 3500 µm.

According to an embodiment the thickness e of the implantable device comprising at least a hydrogel is comprised between 300 and 2500 µm.

According to an embodiment the thickness e of the implantable device comprising at least a hydrogel is comprised between 350 and 2000 µm.

According to an embodiment the thickness e of the implantable device comprising at least a hydrogel is comprised between 400 and 1000 µm.

According to an embodiment, the upper face and the bottom face have each a surface of more than or equal to 1 cm².

According to an embodiment, the upper face and the bottom face have each a surface of more than or equal to 10 cm².

According to an embodiment, the upper face and the bottom face have each a surface of more than or equal to 20 cm².

According to an embodiment, the upper face and the bottom face have each a surface of more than or equal to 50 cm².

By "upper face surface " is meant the surface delimited by the edges of the upper face with the at least one lateral face. The same is true for the bottom face.

According to an embodiment, the upper face and the bottom face have each a surface of less than or equal to 400 cm².

According to an embodiment, the upper face and the bottom face have each a surface of less than or equal to 200 cm².

According to an embodiment, the upper face and the bottom face have each a surface of less than or equal to 100 cm².

According to an embodiment, the upper face and the bottom face have each a surface of less or equal to 50 cm².

According to an embodiment, the implantable device comprising at least a hydrogel has a volume of more than or equal to 10 µl.

According to an embodiment, the implantable device comprising at least a hydrogel has a volume of more than or equal to 50 µl.

According to an embodiment, the implantable device comprising at least a hydrogel has a volume of more than or equal to 100 µl.

According to an embodiment, the implantable device comprising at least a hydrogel has a volume of more than or equal to 200 µl.

According to an embodiment, the implantable device comprising at least a hydrogel has a volume of more than or equal to 500 µl.

According to an embodiment, the implantable device comprising at least a hydrogel has a volume of more than or equal to 1 ml.

According to an embodiment, the implantable device comprising at least a hydrogel has a volume of more than or equal to 2 ml.

According to an embodiment, the implantable device comprising at least a hydrogel has a volume of less than or equal to 20 ml.

According to an embodiment, the implantable device comprising at least a hydrogel has a volume of less than or equal to 10 ml.

According to an embodiment, the implantable device comprising at least a hydrogel has a volume of less than or equal to 5 ml.

According to an embodiment, the implantable device comprising at least a hydrogel has a volume of less than or equal to 2 ml.

According to an embodiment, the implantable device comprising at least a hydrogel has a volume of less than or equal to 1 ml.

In this specification "ml" is considered to be equivalent to cm³.

According to an embodiment, the distance d between two neighbouring channels of the array of channels is less than or equal to 900 µm.

According to an embodiment, the distance d between two neighbouring channels of the array of channels is less than or equal to 800 µm.

According to an embodiment, the distance d between two neighbouring channels of the array of channels is less than or equal to 700 µm.

According to an embodiment, the distance d between two neighbouring channels of the array of channels is less than or equal to 600 µm.

According to an embodiment, the distance d between two neighbouring channels of the array of channels is less than or equal to 500 µm.

According to an embodiment, at least 95 % of the channels have at least one, in particular 2, more particularly 3, or even 4, channels at a distance d of less than or equal to 900 µm.

According to an embodiment, at least 95 % of the channels have at least one, in particular 2, more particularly 3, or even 4, channels at a distance d of less than or equal to 800 µm.

According to an embodiment, at least 95 % of the channels have at least one, in particular 2, more particularly 3, or even 4, channels at a distance d of less than or equal to 700 µm.

According to an embodiment, at least 95 % of the channels have at least one, in particular 2, more particularly 3, or even 4, channels at a distance of less than or equal to 600 µm.

According to an embodiment, at least 95 % of the channels have at least one, in particular 2, more particularly 3, or even 4, channels at a distance of less than or equal to 500 µm.

According to an embodiment, at least 90 % of the channels have at least one, in particular 2, more particularly 3, or even 4, channels at a distance of less than or equal to 900 µm.

According to an embodiment, at least 90 % of the channels have at least one, in particular 2, more particularly 3, or even 4, channels at a distance of less than or equal to 800 µm.

According to an embodiment, at least 90 % of the channels have at least one, in particular 2, more particularly 3, or even 4, channels at a distance of less than or equal to 700 µm.

According to an embodiment, at least 90 % of the channels have at least one, in particular 2, more particularly 3, or even 4, channels at a distance of less than or equal to 600 µm.

According to an embodiment, at least 90 % of the channels have at least one, in particular 2, more particularly 3, or even 4, channels at a distance of less than or equal to 500 µm.

According to an embodiment, at least 95 % of the channels have 3 or more channels at a distance of less than or equal to 900 µm.

According to an embodiment, at least 95 % of the channels have 3 or more channels at a distance of less than or equal to 800 µm.

According to an embodiment, at least 95 % of the channels have 3 or more channels at a distance of less than or equal to 700 µm.

According to an embodiment, at least 95 % of the channels have 3 or more, channel at a distance of less than or equal to 600 µm.

According to an embodiment, at least 95 % of the channels have 3 or more channels at a distance of less than or equal to 500 µm.

According to an embodiment, at least 90 % of the channels have 3 or more channels at a distance of less than or equal to 900 µm.

According to an embodiment, at least 90 % of the channels have 3 or more channels at a distance of less than or equal to 800 µm.

According to an embodiment, at least 90 % of the channels have 3 or more channels at a distance of less than or equal to 700 µm.

According to an embodiment, at least 90 % of the channels have 3 or more, channels at a distance of less than or equal to 600 µm.

According to an embodiment, at least 90 % of the channels have 3 or more channels at a distance of less than or equal to 500 µm.

According to an embodiment, the distance between two neighbouring channels of the array of channels is more than or equal to 150 µm.

According to an embodiment, the distance between two neighbouring channels of the array of channels is more than or equal to 200 µm.

According to an embodiment, the distance between two neighbouring channels of the array of channels is more than or equal to 250 µm.

According to an embodiment, the distance between two neighbouring channels of the array of channels is more than or equal to 300 µm.

According to an embodiment, the distance between two neighbouring channels of the array of channels is comprised between 150 to 1000 µm.

According to an embodiment, the distance between two neighbouring channels of the array of channels is comprised between 200 to 1000 µm.

According to an embodiment, the distance between two neighbouring channels of the array of channels is comprised between 250 to 800 µm.

According to an embodiment, the distance between two neighbouring channels of the array of channels is comprised between 300 to 600 µm.

According to an embodiment, the distance between at least 95 % of the channels have at least one channel at a distance comprised between 150 to 1000 µm.

According to an embodiment, at least 95 % of the channels have at least one, in particular 2, more particularly 3, or even 4, channels at a distance comprised between 250 to 800 µm.

According to an embodiment, at least 95 % of the channels have at least one, in particular 2, more particularly 3, or even 4, channels at a distance comprised between 300 to 600 µm.

According to an embodiment, at least 95 % of the channels have 3 or more channels at a distance comprised between 250 to 800 µm.

According to an embodiment, at least 95 % of the channels have 3 or more channels at a distance comprised between 300 to 600 µm.

According to an embodiment, the implantable device comprising at least a hydrogel comprises at least one channel of the array of channels which is at a distance of at least one lateral face of more than or equal to 150 µm.

According to an embodiment, the implantable device comprising at least a hydrogel comprises at least one channel of the array of channels which is at a distance of at least one lateral face of more than or equal to 200 µm.

According to an embodiment, the implantable device comprising at least a hydrogel comprises at least one channel of the array of channels which is at a distance of at least one lateral face of more than or equal to 250 µm.

According to an embodiment, the implantable device comprising at least a hydrogel comprises at least one channel of the array of channels which is at a distance of at least one lateral face of more than or equal to 300 µm.

In an embodiment the hydrogel comprises an array of channels that includes:
- central channels,
- peripheral channels which are closer to the at least one lateral face than the central channels.

By "peripheral channels" is meant channel at a distance of the edge of the at least one lateral face of less or equal to 1000 µm.

According to an embodiment, the hydrogel comprises peripheral channels at a distance of less than or equal to 800 µm of the at least one lateral face.

According to an embodiment, the hydrogel comprises peripheral channels at a distance of less than or equal to 700 µm of the at least one lateral face.

According to an embodiment, the hydrogel comprises peripheral channels at a distance of less than or equal to 600 µm of the at least one lateral face.

According to an embodiment, the distance between each peripheral channels at a distance of less than or equal to 500 µm of the at least one lateral face.

In an embodiment, the hydrogel comprises at least one peripheral channel per 5 mm of the at least one lateral face.

In an embodiment, the hydrogel comprises at least one peripheral channel per 3 mm of the at least one lateral face.

In an embodiment, the hydrogel comprises at least one peripheral channel per 2 mm of the at least one lateral face.

In an embodiment, the hydrogel comprises at least one peripheral channel per 1 mm of the at least one lateral face.

According to an embodiment, at least one channel of the array of channels consists in a through hole extending between the upper and the bottom faces.

According to an embodiment, at least 50 % of the channels of the array of channels are through holes extending between the upper and the bottom faces.

According to an embodiment, at least 75 % of the channels of the array of channels are through holes extending between the upper and the bottom faces.

According to an embodiment, 90 % of the channels of the array of channels are through holes extending between the upper and the bottom faces.

According to an embodiment, all the channels of the array of channels are through holes extending between the upper and the bottom faces.

According to an embodiment, at least one channel of the array of channels consists in a blind hole.

According to an embodiment, at least 50 % of the channels of the array of channels are blind holes.

According to an embodiment, at least 75 % of the channels of the array of channels are blind holes.

According to an embodiment, at least 90 % of the channels of the array of channels are blind holes.

According to an embodiment, all the channels of the array of channels are blind holes.

According to an embodiment at least two channels of the array of channels consist in blind holes:
- a first blind hole extending from the upper face, and
- a second blind hole extending from the bottom face.

According to an embodiment, the implantable device comprising at least a hydrogel comprises blind holes, said blind holes being located in the upper face or in the bottom face, the ratio between the blind holes located in the upper face and the blind holes located in the bottom face being comprised between 90/10 to 10/90, in particular between 75/20 to 25/75, more particularly between 3/1 to 1/3, even more particularly between 60/40 to 40/60.

According to an embodiment, the implantable device comprising at least a hydrogel comprises first blind holes on the uppoer face and second blind holes on the bottom face which are offset relative to one another according to a direction perpendicular to the thickness e of the implantable device.

According to an embodiment, the surface of each of the opening of the blind holes and through holes is at least of 1 500 µm².

According to an embodiment, the surface of each of the opening of the blind holes and through holes is at least of 10 000 µm².

According to an embodiment, the surface of each of the opening of the blind holes and through holes is at least of 50 000 µm².

According to an embodiment, the surface of each of the opening of the blind holes and through holes is at least of 100 000 µm².

According to an embodiment, the surface of each of the opening of the blind holes and through holes is at least of 150 000 µm².

According to an embodiment, the surface of each of the opening of the blind holes and through holes is at most of 250 000 µm².

According to an embodiment, the surface of each of the opening of the blind holes and through holes is at most of 500 000 µm².

According to an embodiment, the surface of each of the opening of the blind holes and through holes is at most of 1 000 000 µm².

According to an embodiment, the implantable device comprising at least a hydrogel comprises at least one channel of the array of channels consisting in a groove extending transversely according to a direction perpendicular to the thickness e of the implantable device.

According to an embodiment, the blind holes q and the grooves g have a depth H"of more than or equal to 100 µm.

According to an embodiment, the blind holes q and the grooves g have a depth H"of more than or equal to 200 µm.

According to an embodiment, the blind holes q and the grooves g have a depth H" of more than or equal to 300 µm.

According to an embodiment, the blind holes q and the grooves g have a depth H" of more than or equal to 400 µm.

According to an embodiment, the blind holes q and the grooves g have a depth H" of more than or equal to 500 µm.

According to an embodiment, the blind holes q and the grooves g have a depth H"of more than or equal to 700 µm.

According to an embodiment, the blind holes q and the grooves g have a depth H" of more than or equal to 30 % of the thickness e of the implantable device comprising at least a hydrogel.

According to an embodiment, the blind holes q and the grooves g have a depth H" of more than or equal to 40 % of the thickness e of the implantable device comprising at least a hydrogel.

According to an embodiment, the blind holes q and the grooves g have a depth H"of more than or equal to 50 % of the thickness e of the implantable device comprising at least a hydrogel.

According to an embodiment, the blind holes q and the grooves g have a depth H"of more than or equal to 60 % of the thickness e of the implantable device comprising at least a hydrogel.

According to an embodiment, the blind holes q and the grooves g have a depth H"of more than or equal to 70 % of the thickness e of the implantable device comprising at least a hydrogel.

According to an embodiment, the blind holes q and the grooves g have a depth H"of more than or equal to 80 % of the thickness e of the implantable device comprising at least a hydrogel.

According to an embodiment, the blind holes q and the grooves g have a depth H"of more than or equal to 90 % of the thickness e of the implantable device comprising at least a hydrogel.

According to an embodiment, the bottom of the blind holes g and of the grooves g is at a distance of at most 300 µm of the opposite face.

According to an embodiment, the bottom of the blind holes g and of the grooves g is at a distance of at most 400 µm of the opposite face.

According to an embodiment, the bottom of the blind holes g and of the grooves g is at a distance of at most 500 µm of the opposite face.

According to an embodiment, the bottom of the blind holes g and of the grooves g is at a distance of at least 100 µm of the opposite face.

According to an embodiment, the bottom of the blind holes g and of the grooves g is at a distance of at least 200 µm of the opposite face.

According to an embodiment, the bottom of the blind holes g and of the grooves g is at a distance of at least 300 µm of the opposite face.

According to an embodiment, the openings of the channels represent at least 2 % of the total surface of the implantable device comprising at least a hydrogel.

According to an embodiment, the openings t" of the channels represent at least 4 % of the total surface of the implantable device comprising at least a hydrogel.

According to an embodiment, the openings t" of the channels represent at least 5 % of the total surface of the implantable device comprising at least a hydrogel.

According to an embodiment, the openings t" of the channels represent at least 8 % of the total surface of the implantable device comprising at least a hydrogel.

According to an embodiment, the openings t" of the channels represent at least 10 % of the total surface of the implantable device comprising at least a hydrogel.

By total surface of the implantable device comprising at least a hydrogel is meant the sum of the planar surface delimited by the edges of the upper face and of the planar surface delimited by the edges of the bottom face.

As an example the surface of the opening of a channel which is a round blind hole is pi x (radius of the circle)².

As an example the surface of the 2 openings of a channel which is a round through hole is 2 x pi x (radius of the circle)², as it has an opening on the upper face and on the bottom face.

According to an embodiment, the implantable device comprising at least a hydrogel according to the invention has at least 80 %, 85%, 90%, 95%, 98%, 99%, 100% of its volume at most at 1 µm of the closest surface / interface between the hydrogel and the outside of the hydrogel.

According to an embodiment, the implantable device comprising at least a hydrogel according to the inventionhas at least 80 %, 85%, 90%, 95%, 98%, 99%, 100% of its volume at most at 750 nm of the closest surface / interface between the hydrogel and the outside of the hydrogel.

According to an embodiment, the implantable device comprising at least a hydrogel according to the inventionhas at least 80 %, 85%, 90%, 95%, 98%, 99%, 100% of its volume at most at 500 nm of the closest surface / interface between the hydrogel and the outside of the hydrogel.

According to an embodiment, the implantable device comprising at least a hydrogel according to the inventionhas at least 80 %, 85%, 90%, 95%, 98%, 99%, 100% of its volume at most at 300 nm of the closest surface / interface between the hydrogel and the outside of the hydrogel.

According to an embodiment, the implantable device comprising at least a hydrogel according to the inventionhas at least 80 %, 85%, 90%, 95%, 98%, 99%, 100% of its volume at most at 250 nm of the closest surface / interface between the hydrogel and the outside of the hydrogel.

According to an embodiment, the implantable device comprising at least a hydrogel according to the inventionhas at least 80 %, 85%, 90%, 95%, 98%, 99%, 100% of its volume at most at 200 nm of the closest surface / interface between the hydrogel and the outside of the hydrogel.

According to an embodiment, the depth of the channels is at least 50%, 60%, 70%, 80%, 90% or even 100% of the thickness e of the hydrogel.

According to an embodiment, the the depth of the channels is at least 100 µm, 200µm, 300 µm, 400 µm, 500 µm, 600 µm, 700 µm, 800 µm, 900 µm, 1000 µm .

According to an embodiment, the the bottom of the channels is at least 100 µm, 200µm, 300 µm, 400 µm, 500 µm from the face of the implantable device comprising at least a hydrogel.

According to an embodiment, the height of the channel is at least 40 %, 50 %, 60 %, 70%, 80 %, 90 % or even 95 % of the thickness e of the hydrogel .

According to an embodiment, the channels are cavities or grooves .

According to an embodiment, the cavities are circular.

According to an embodiment, the cavities have a diameter ø comprised between 50 to 1000 µm, 100 to 500 µm, 250 to 500 µm.

According to an embodiment, the grooves have a width comprised between 50 to 1000 µm, 100 to 500 µm, 250 to 500 XX µm

According to an embodiment, the grooves have a ratio length/width of at least 2, 5, 10, 20, 50. They may have a length of at least 1 mm, 5 mm, 1 cm, 2 cm, 5 cm, 10 cm.

According to an embodiment, the channels are going through the hydrogel, in particular they are holes or lumen.

According to an embodiment, the holes or lumen are circular, in particular they may have a diameter ø comprised between 50 to 1000 µm, 100 to 500 µm, 250 to 500 µm.

According to an embodiment, the channels allow the hydrogel to increase its surface / volume ratio by at least 1%, 2%, 3%, 4%, 5%, 10%,

According to an embodiment, the channels are geometrically placed, in particular in rows, parallels, quincuncially.

According to an embodiment, the implantable device comprising at least a hydrogel is rectangular shaped, square shaped or disc shaped, in particular the disc shape is circular or oval.

In an embodiment the hydrogel comprises polymers which are crosslinked via covalent bonding.

The properties of this family of hydrogels are tunable and tailorisable to the applications by choosing and adapting the crosslinking reaction conditions, the substitution degree and molecular weight of the dextrans and crosslinkers.

In an embodiment the polymer is a dextran polymer bearing carboxylate groups.

In an embodiment the crosslinker is at least one at least divalent radical L(-)ᵢ, this at least divalent radical being a linear, branched or cyclic alkyl radical comprising at least 15 carbon atoms and optionally heteroatoms such as oxygen, nitrogen or sulfur.

In an embodiment, the hydrogel is a crosslinked dextran polymer, bearing carboxylate groups, wherein at least two saccharidic units of dextran belonging to two different polymer chains are covalently linked by at least one at least divalent radical L(-)ᵢ, this at least divalent radical being a linear, branched or cyclic alkyl radical comprising at least 15 carbon atoms and optionally heteroatoms such as oxygen, nitrogen or sulfur.

In an embodiment, the crosslinked dextran polymer is a dextran polymer bearing carboxylate groups wherein the at least divalent radical -L(-)ᵢ is covalently bound to the dextran polymer backbone with i -(R₁)ₘG₁- - radicals, wherein,
- L(-)ᵢ is a linear or branched polyether bearing at its ends, heteroatoms such as oxygen, nitrogen or sulfur,
- i is the valence of L and the number of -(R₁)ₘG₁- - radicals and is an integer comprised from 2 to 8 (2 ≤ i ≤ 8)
- m is an integer equal to 0 or 1,
- -R₁- is a linear or branched alkyl divalent radical comprising from 1 to 6 carbon atoms and optionally heteroatoms such as oxygen, nitrogen or sulfur.
- -G₁- is a linear or branched or cyclic alkyl divalent radical comprising from 1 to 6 carbon atoms and may comprise heteroatoms such as oxygen, nitrogen or sulfur.

In an embodiment, when m is 1, -R₁- is linked to the dextran and -G₁- is linked to the linker.

In an embodiment, the crosslinked dextran polymer is a dextran polymer wherein L(-)ᵢ is a linear polyether radical bearing at its ends, at least two heteroatoms such as oxygen, nitrogen or sulfur,

In an embodiment, the crosslinked dextran polymer is a dextran polymer wherein L(-)ᵢ is a branched polyether radical bearing at its ends, at least two heteroatoms such as oxygen, nitrogen or sulfur.

By branched polyether is meant several polyether arms connected by a linker. The linker may be an alkyl comprising between 2 to 20 carbon atoms, and optionally heteroatoms such as oxygen, nitrogen or sulfur.

In an embodiment, the crosslinked dextran polymer is a dextran polymer wherein L(-)ᵢ is a linear or branched polyether bearing at its ends, at least two heteroatoms such as oxygen, nitrogen or sulfur comprising at most 8 arms.

In an embodiment, the crosslinked dextran polymer is a dextran polymer wherein L(-)ᵢ with i = 2 is a linear or branched polyether bearing at its ends, 2 heteroatoms such as oxygen, nitrogen or sulfur comprising 2 arms.

In an embodiment, the crosslinked dextran polymer is a dextran polymer, wherein L(-)ᵢ with i = 3 is a branched polyether bearing at its ends, 3 heteroatoms such as oxygen, nitrogen or sulfur comprising 3 arms.

In an embodiment, the crosslinked dextran polymer is a dextran polymer, wherein L(-)ᵢ with i = 4 is a branched polyether bearing at its ends, 4 heteroatoms such as oxygen, nitrogen or sulfur comprising 4 arms.

In an embodiment, the crosslinked dextran polymer is a dextran polymer wherein L(-)ᵢ with i = 5 is a branched polyether bearing at its ends, 5 heteroatoms such as oxygen, nitrogen or sulfur comprising 5 arms.

In an embodiment, the crosslinked dextran polymer is a dextran polymer whereinL(-)ᵢ with i = 6 is a branched polyether bearing at its ends, 6 heteroatoms such as oxygen, nitrogen or sulfur comprising 6 arms.

In an embodiment, the crosslinked dextran polymer is a dextran polymer wherein L(-)ᵢ with i = 7 is a branched polyether bearing at its ends, 7 heteroatoms such as oxygen, nitrogen or sulfur comprising 7 arms.

In an embodiment, the crosslinked dextran polymer is a dextran polymer wherein L(-)ᵢ with i = 8 is a branched polyether bearing at its ends, 8 heteroatoms such as oxygen, nitrogen or sulfur comprising 8 arms

In an embodiment, the crosslinked dextran polymer is a dextran polymer wherein L(-)ᵢ with i = 2 is a linear polyether bearing at its ends 2 sulfur atoms.

In an embodiment, the crosslinked dextran polymer is a dextran polymer wherein L(-)ᵢ with i = 4 is a branched polyether bearing at its ends at most 4 sulfur atoms.

In an embodiment, the crosslinked dextran polymer is a dextran polymer wherein L(-)ᵢ is a branched polyether bearing at its ends a sulfur atom and comprising at most 8 arms.

In an embodiment, the crosslinked dextran polymer is a dextran polymer wherein L(-)ᵢ is a branched polyether bearing at its ends 2 sulfur atoms and comprising 2 arms.

In an embodiment, the crosslinked dextran polymer is a dextran polymer wherein L(-)ᵢ with i = 3 is a branched polyether bearing at its ends 3 sulfur atoms and comprising 3 arms.

In an embodiment, the crosslinked dextran polymer is a dextran polymer wherein L(-)ᵢ with i = 4 is a branched polyether bearing at its ends 4 sulfur atoms and comprising 4 arms.

In an embodiment, the crosslinked dextran polymer is a dextran polymer wherein L(-)ᵢ with i = 8 - is a branched polyether bearing at its ends 8 sulfur atoms and comprising 8 arms.

In an embodiment, the crosslinked dextran polymer is a dextran polymer wherein L(-)ᵢ is a radical issued from a linear or branched mercaptopolyethyleneglycol comprising at least 2 sulfur atoms and comprising at most 8 arms, which
- number-average molecular weight (Mn) is comprised from 500 to 40 000 g/mol (500 ≤ Mn ≤ 40 000 g/mol) or
- polymerisation degree (DP) is comprised from 8 to 1000 (8 ≤ DP ≤ 1000).

In an embodiment, the crosslinked dextran polymer is a dextran polymer wherein L(-)ᵢ is a radical issued from a linear or branched mercaptopolyethyleneglycol comprising at least two sulfur atoms and comprising at most 8 arms, which
- Mn is comprised from 1000 to 25 000 g/mol (1000 ≤ Mn ≤ 25 000 g/mol) or
- polymerisation degree is comprised from 15 to 600 (15 ≤ DP ≤ 600).

In an embodiment, the crosslinked dextran polymer is a dextran polymer wherein L(-)ᵢ is a radical issued from a mercaptopolyethyleneglycol according to formula II:

-S-(CH₂-CH₂-O)ₚ-CH₂-CH₂-S- Formula II

wherein p is an integer comprised from 8 to 1000 (8 ≤ p ≤ 1000).

In an embodiment, the cross-linked dextran polymer according to the invention is a dextran polymer wherein L(-)ᵢ is a radical according to formula III' issued from a mercaptoethyl polyoxyethylene: 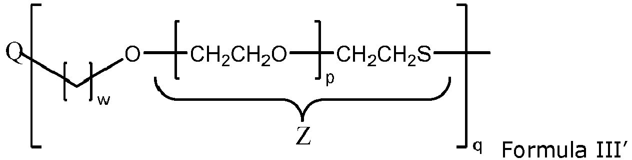 wherein
- Q is either a carbon atom or an alkyl chain comprising 2 to 10 carbon atoms , the alkyl chain may comprise heteroatom chosen from the group consisting of oxygen, sulfur and nitrogen
- p is an integer comprised from 8 to 1000 (8 ≤ p ≤ 1000)
- q is an integer comprised from 2 to 8 (2 ≤ q ≤ 8)
- w is 1 or 2, and
- Z is -(CH₂-CH₂O)ₚ-CH₂-CH₂-S-.

In an embodiment, w is 1.

In an embodiment, the crosslinked dextran polymer is a dextran polymer wherein L(-)ᵢ is a radical according to formula III issued from a mercaptoethyl polyoxyethylene: 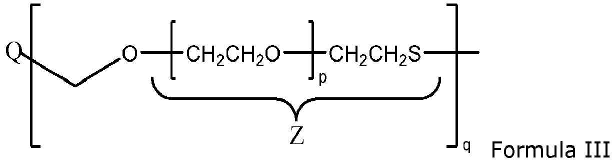 wherein
- Q is either a carbon atom or an alkyl chain comprising from 2 to 10 carbon atoms , the alkyl chain may comprise heteroatom chosen from the group consisting of oxygen, sulfur and nitrogen
- p is an integer comprised from 8 to 1000 (8 ≤ p ≤ 1000)
- q is an integer comprised form 2 to 8 (2 ≤ q ≤ 8)
- Z is -(CH₂-CH₂-O)ₚ-CH₂-CH₂-S-.

In an embodiment Q is an alkyl chain comprising from 2 to 8 carbon atoms and 1 or 2 oxygen atoms.

In an embodiment, the cross-linked dextran polymer according to the invention is a dextran polymer wherein L(-)ᵢ corresponding to Formula III' with q = 4 and Q is a carbon atom, and is a radical according to formula VII 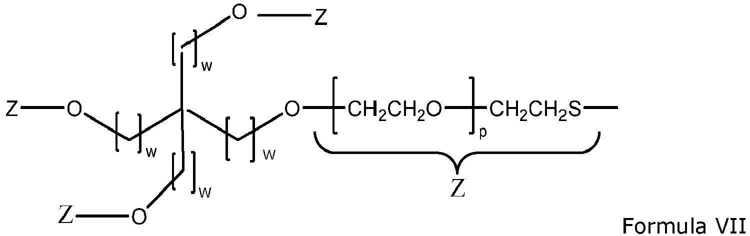 wherein
- Z is -(CH₂-CH₂O)ₚ-CH₂-CH₂-S-
- p is comprised from 8 to 1000 (8 ≤ p ≤ 1000)
- w is comprised from 1 to 2, (1 ≤ w ≤ 2).

In an embodiment, w is 1.

In an embodiment, the crosslinked dextran polymer according to the invention is a dextran polymer wherein L(-)ᵢ is a radical according to formula VII'

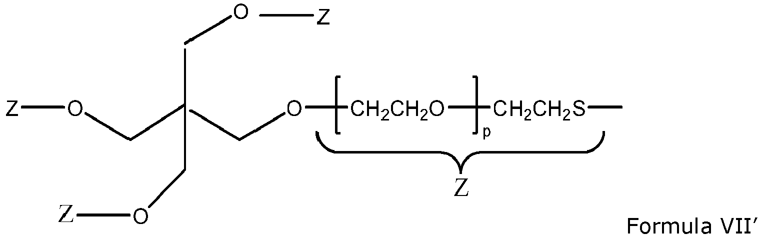

Wherein:
- p is comprised from 8 to 1000, (8 ≤ p ≤ 1000) and
- Z is -(CH₂-CH₂O)ₚ-CH₂-CH₂-S-.

In an embodiment, the cross-linked dextran polymer according to the invention is a dextran polymer wherein L(-)ᵢ is a radical according to formula II, III, III', VII or VII' issued from the thiol polyethylene glycols or mercaptopolyoxyethylenes cited in the following table:

| Chemical Name | i | Mn (kg/mol) |
|---|---|---|
| Poly(ethylene glycol) dithiol | 2 | 10 |
| Poly(ethylene glycol) dithiol | 2 | 3.4 |
| Poly(ethylene glycol) dithiol | 2 | 1 |
| Pentaerythritol tetra(mercaptoethyl) polyoxyethylene | 4 | 5.2 |
| Pentaerythritol tetra(mercaptoethyl) polyoxyethylene | 4 | 20 |
| tripentaerythritol octa(mercaptoethyl) polyoxyethylene | 8 | 20 |

In an embodiment, the cross-linked dextran polymer according to the invention is a dextran polymer wherein L(-)ᵢ is a radical according to formula VII with w = 1 or formula VII' in particular issued from a pentaerythritol tetra(mercaptoethyl) polyoxyethylene, CAS# 188492-68-4.

In an embodiment, the cross-linked dextran polymer according to the invention is a dextran polymer wherein -R₁- is a linear or branched alkyl divalent radical comprising from 1 to 6 carbon atoms and optionally heteroatoms such as oxygen, nitrogen or sulfur .

In an embodiment, the cross-linked dextran polymer according to the invention is a dextran polymer wherein -R₁- is a linear or branched alkyl divalent radical comprising a nitrogen atom.

In an embodiment, the cross-linked dextran polymer according to the invention is a dextran polymer wherein -R₁-, is a linear alkyl radical comprising from 1 to 6 carbon atoms and a nitrogen atom.

In an embodiment, the cross-linked dextran polymer according to the invention is a dextran polymer wherein -R₁-, is a linear alkyl radical comprising from 2 to 5 carbon atoms and a nitrogen atom.

In an embodiment, the cross-linked dextran polymer according to the invention is a dextran polymer wherein -R₁-, is a linear alkyl radical comprising from 3 to 4 carbon atoms and a nitrogen atom.

In an embodiment, the cross-linked dextran polymer according to the invention is a dextran polymer wherein -R₁-, is a linear alkyl radical comprising 2 carbon atoms and a nitrogen atom.

In an embodiment, the cross-linked dextran polymer according to the invention is a dextran polymer wherein -R₁-, is a linear alkyl radical comprising from 2 to 5 carbon atoms and a nitrogen atom according to formula XIII:

-NHCH₂CH₂- Formula XIII

In an embodiment, the cross-linked dextran polymer according to the invention is a dextran polymer wherein radical -R₁- is covalently bound by an amide function resulting of the reaction of a carboxylate group borne by the dextran with one -R₁₋ precursor or -(R₁)ₘG₁- precursor bearing an amine function.

In an embodiment, the cross-linked dextran polymer according to the invention is a dextran polymer wherein radical -R₁- is covalently bound by an ether function resulting of the reaction of the hydroxyl function borne by the dextran with one -R₁₋ precursor or -(R₁)ₘG₁- precursor bearing a leaving group, in particular a halogen atom.

In an embodiment, the cross-linked dextran polymer according to the invention is a dextran polymer wherein the radical -G₁- is a divalent radical according to formula V: 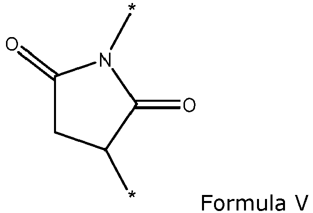 wherein the -^{∗} represent the attachment sites to the dextran backbone and to the linker.

In an embodiment, the cross-linked dextran polymer according to the invention is a dextran polymer wherein the radical -G₁- is a radical according to formula V':

-S-CH2-SO2-CH2-CH2- Formula V'

In an embodiment, the cross-linked dextran polymer according to the invention is a dextran polymer wherein -(R₁)ₘG₁- covalently bound to a carboxylate group borne by the dextran polymer backbone is a divalent radical according to formula VI: 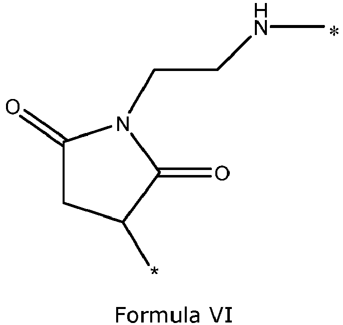 wherein the -^{∗} represent the attachment sites to the dextran backbone and to the linker.

In an embodiment, the cross-linked dextran polymer according to the invention is a dextran polymer wherein -(R₁)ₘG₁- covalently bound to a carboxylate group borne by the dextran polymer backbone is a divalent radical according to formula VIII: 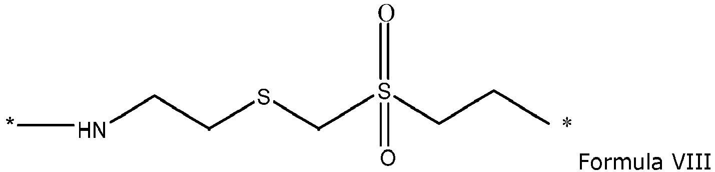 wherein the -^{∗} represent the attachment sites to the dextran backbone and to the linker.

In an embodiment, the cross-linked dextran polymer according to the invention is a dextran polymer wherein -(R₁)ₘG₁- covalently bound to a hydroxyl function borne by the dextran polymer backbone is a divalent radical according to formula X: 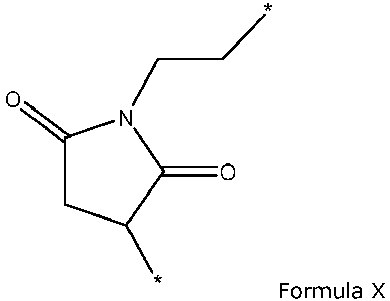 wherein the -^{∗} represent the attachment sites to the dextran backbone and to the linker.

In an embodiment, the cross-linked dextran polymer according to the invention is a dextran polymer wherein -(R₁)ₘG₁- covalently bound to a hydroxyl function borne by the dextran polymer backbone is a divalent radical according to formula XI: 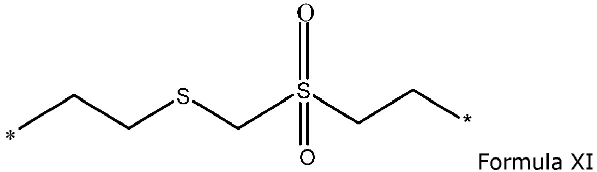 wherein the -^{∗} represent the attachment sites to the dextran backbone and to the linker.

In an embodiment in Formulas VI and X, -(R₁)ₘG₁- is linked to the dextran via the upper bond ie via R₁ or N-ethyl bond and to the linker via the lower bond, ie the bond from the saturated ring.

In an embodiment in Formulas VIII and XI, -(R₁)ₘG₁- is linked to the dextran via the left bond ie via R₁ and to the linker via the right bond.

In an embodiment, the cross-linked dextran polymer according to the invention is a dextran polymer wherein the carboxylate groups bound to the saccharidic units are bound by ether bonds and chosen amongst linear or branched alkyl radicals bearing at least a carboxylate group.

In an embodiment, the cross-linked dextran polymer according to the invention is a dextran polymer wherein carboxylate groups bound to the saccharidic units are bound by ether bonds and chosen amongst carboxylate groups according to formula I

-(CH₂)ₙ-COX Formula I

wherein
- n is an integer comprised from 1 to 7 (1 ≤ n ≤ 7)
- X is chosen amongst -OH, -ONa, -OK or -(R₁)ₘG₁- radical.

In an embodiment, the cross-linked dextran polymer according to the invention is a dextran polymer wherein -(CH₂)ₙ-COX is covalently bound to the dextran polymer backbone by an ether function resulting of the reaction of the hydroxyl function borne by the dextran with one -(CH₂)ₙ-COX precursor bearing a leaving group, in particular a halogen atom.

In an embodiment, the cross-linked dextran polymer according to the invention is a dextran polymer wherein -(CH₂)ₙ-COX which is not bound to -(R₁)ₘG₁- radical is in a salt form and X is chosen amongst ONa or OK.

In an embodiment, the cross-linked dextran polymer according to the invention is a dextran polymer bearing carboxylate groups according to formula I wherein n is comprised from 1 to 7.

In an embodiment, the cross-linked dextran polymer according to the invention is a dextran polymer bearing carboxylate groups according to formula I wherein n is comprised from 1 to 6.

In an embodiment, the cross-linked dextran polymer according to the invention is a dextran polymer bearing carboxylate groups according to formula I wherein n is comprised from 1 to 5.

In an embodiment, the cross-linked dextran polymer according to the invention is a dextran polymer bearing carboxylate groups according to formula I wherein n is comprised from 1 to 4.

In an embodiment, the cross-linked dextran polymer according to the invention is a dextran polymer bearing carboxylate groups according to formula I wherein n is comprised from 1 to 3.

In an embodiment, the cross-linked dextran polymer according to the invention is a dextran polymer bearing carboxylate groups according to formula I wherein n is comprised from 1 to 2.

In an embodiment, the cross-linked dextran polymer according to the invention is a dextran polymer bearing carboxylate groups according to formula I wherein n is 1.

In an embodiment, the cross-linked dextran polymer according to the invention is a dextran polymer wherein the dextran polymer backbone is according to formula XII, 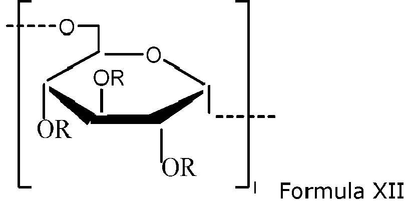 wherein R is chosen among
- -H, -(CH₂)ₙ-COX or -(R₁)ₘG₁-; n, m, X, -R₁-, -G₁- and L(-)i being defined as above and L(-)i being covalently bound to another dextran polymer backbone with a -(R₁)ₘG₁- radical, and
- I is comprised from 20 to 5000 (20 ≤ I ≤ 5000)

In an embodiment, the cross-linked dextran polymer according to the invention is a dextran polymer wherein the dextran polymer backbone is a dextran having a weight average molecular weight (Mw) comprised from 5 to 1000 kDa before cross-linking and substitution.

In other words, the cross-linked dextran polymer according to the invention is obtained after substitution and crosslinking of a native dextran polymer having a weight average molecular weight (Mw) comprised from 5 to 1000 kDa.

In an embodiment, the cross-linked dextran polymer according to the invention is a dextran polymer wherein the dextran polymer backbone is a dextran having a weight average molecular weight (Mw) comprised from 5 to 250 kDa before cross-linking and substitution.

In an embodiment, the cross-linked dextran polymer according to the invention is a dextran polymer wherein the dextran polymer backbone is a dextran having a weight average molecular weight (Mw) comprised from 5 to 100 kDa before cross-linking and substitution.

In an embodiment, the cross-linked dextran polymer according to the invention is a dextran polymer wherein the dextran polymer backbone is a dextran having a weight average molecular weight (Mw) comprised from 5 to 50 kDa before cross-linking and substitution.

In an embodiment, the cross-linked dextran polymer according to the invention is a dextran polymer wherein the dextran polymer backbone is a dextran having a weight average molecular weight (Mw) comprised from 5 to 25 kDa before cross-linking and substitution.

In an embodiment, the cross-linked dextran polymer according to the invention is a dextran polymer wherein the dextran polymer backbone is a dextran having a weight average molecular weight (Mw) comprised from 250 to 1000 kDa before cross-linking and substitution.

In an embodiment, the cross-linked dextran polymer according to the invention is a dextran polymer wherein the dextran polymer backbone is a dextran having a weight average molecular weight (Mw) comprised from 10 to 500 kDa before cross-linking and substitution.

In an embodiment, the cross-linked dextran polymer according to the invention is a dextran polymer wherein the dextran polymer backbone is a dextran having a weight average molecular weight (Mw) comprised from 20 to 500 kDa before cross-linking and substitution.

In an embodiment, the cross-linked dextran polymer according to the invention is a dextran polymer wherein the dextran polymer backbone is a dextran having a weight average molecular weight (Mw) comprised from 20 to 100 kDa before cross-linking and substitution.

In an embodiment, the cross-linked dextran polymer according to the invention is a dextran polymer wherein the dextran polymer backbone is a dextran having a weight average molecular weight (Mw) comprised from 20 to 50 kDa before cross-linking and substitution.

In an embodiment, the cross-linked dextran polymer according to the invention is a dextran polymer wherein the dextran polymer backbone is a dextran having a weight average molecular weight (Mw) comprised from 40 to 250 kDa before cross-linking and substitution.

In an embodiment, the cross-linked dextran polymer according to the invention is a dextran polymer wherein the dextran polymer backbone is a dextran having a weight average molecular weight (Mw) comprised from 40 to 100 kDa before cross-linking and substitution.

In an embodiment, the cross-linked dextran polymer according to the invention is a cross-linked dextran polymer wherein the degree of substitution (DS₁) of the dextran backbone with the -(R₁)ₘG₁- groups is comprised in the range from 0.001 to 0.4 (0.001 ≤ DS₁ ≤ 0.4).

In an embodiment, the cross-linked dextran polymer according to the invention is a cross-linked dextran polymer wherein the degree of substitution (DS₁) of the dextran backbone with the -(R₁)ₘG₁- groups is comprised in the range from 0.01 to 0.4 (0.01 ≤ DS₁ ≤ 0.4).

In an embodiment, the cross-linked dextran polymer according to the invention is a cross-linked dextran polymer wherein the degree of substitution (DS₁) of the dextran backbone with the -(R₁)ₘG₁- groups is comprised in the range from 0.1 to 0.4 (0.1 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 5 to 250 kDa before cross-linking and substitution and the degree of substitution (DS₁) of the dextran backbone with the -(R₁)ₘG₁- groups is comprised in the range from 0.1 to 0.4 (0.1 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 20 to 100 kDa before cross-linking and substitution and the degree of substitution (DS₁) of the dextran backbone with the -(R₁)mG₁- groups is comprised in the range from 0.2 to 0.4 (0.2 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 20 to 100 kDa before cross-linking and substitution and the degree of substitution (DS₁) of the dextran backbone with the -(R₁)mG₁- groups is comprised in the range from 0.2 to 0.3 (0.2 ≤ DS₁ ≤ 0.3).

In an embodiment, the cross-linked dextran polymer according to the invention is a cross-linked dextran polymer wherein the dextran polymer backbone is a dextran having a weight average molecular weight (Mw) is from 250 to 1000 kDa before cross-linking and substitution and the degree of substitution (DS₁) of the dextran backbone with the -(R₁)ₘG₁- groups is comprised in the range from 0.001 to 0.4 (0.001 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before cross-linking and substitution and the degree of substitution (DS₁) of the dextran backbone with the -(R₁)ₘG₁- groups is comprised in the range from 0.01 to 0.4 (0.01 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before cross-linking and substitution and the degree of substitution (DS₁) of the dextran backbone with the -(R₁)ₘG₁- groups is comprised in the range from 0.1 to 0.4 (0.1 ≤ DS₁ ≤ 0.4).

In an embodiment, the cross-linked dextran polymer according to the invention is a cross-linked dextran polymer wherein the degree of substitution (DS₂) of the dextran backbone with the carboxylate groups according to formula I is comprised in the range from 0.5 to 3 (0.5 ≤ DS₂ ≤ 3).

In an embodiment, the cross-linked dextran polymer according to the invention is a cross-linked dextran polymer wherein the degree of substitution (DS₂) of the dextran backbone with the carboxylate groups according to formula I is comprised in the range from 1 to 2.75 (1 ≤ DS₂ ≤ 2.75).

In an embodiment, the cross-linked dextran polymer according to the invention is a cross-linked dextran polymer wherein the degree of substitution (DS₂) of the dextran backbone with the carboxylate groups according to formula I is comprised in the range from 1.5 to 2.5 (1.5 ≤ DS₂ ≤ 2.5).

In an embodiment, the cross-linked dextran polymer according to the invention is a cross-linked dextran polymer wherein the degree of substitution (DS₂) of the dextran backbone with the carboxylate groups according to formula I is comprised in the range from 1.75 to 2.25 (1.75 ≤ DS₂ ≤ 2.25).

In an embodiment, the cross-linked dextran polymer according to the invention is a cross-linked dextran polymer having a molar ratio (DC) between the molar concentration of the -(R₁)ₘG₁- radical and the molar concentration of the reactive functions of the cross-linker L(-)ᵢ comprised in a range from 0.5 to 1.5 (0.5 ≤ DC ≤ 1.5).

In an embodiment, the cross-linked dextran polymer according to the invention is a cross-linked dextran polymer having a molar ratio between the molar concentration of the -(R₁)mG₁- radical and the molar concentration of the reactive functions of the cross-linker L(-)ᵢ is comprised in a range from 0.8 to 1.2 (0.8 ≤ DC ≤ 1.2).

In an embodiment, the cross-linked dextran polymer according to the invention is a cross-linked dextran polymer having a molar ratio between the molar concentration of the -(R₁)mG₁- radical and the molar concentration of the reactive functions of the cross-linker L(-)ᵢ is comprised in a range from 0.9 to 1.1 (0.9 ≤ DC ≤ 1.1).

In an embodiment, the cross-linked dextran polymer according to the invention is a cross-linked dextran polymer having a molar ratio between the molar concentration of the -(R₁)mG₁- radical and the molar concentration of the reactive functions of the cross-linker L(-)ᵢ is comprised in a range from 0.95 to 1.05 (0.95 ≤ DC ≤ 1.05).

In an embodiment, the cross-linked dextran polymer according to the invention is a cross-linked dextran polymer having a molar ratio between the molar concentration of the -(R₁)mG₁- radical and the molar concentration of the reactive functions of the cross-linker L(-)ᵢ is 1 (DC = 1).

The invention also concerns a hydrogel comprising the cross-linked dextran polymer according to the invention.

In an embodiment the hydrogel according to the invention is characterized in that Tan δ is lower than 1.

In the present specification, tan δ is the ratio of the storage modulus (also called elastic modulus) G' to the loss modulus G" (tan δ = G'/G").

In an embodiment the hydrogel according to the invention is characterized in that Tan δ is less than or equal to 0.5.

In an embodiment the hydrogel according to the invention is characterized in that Tan δ is less than or equal to 0.1.

In an embodiment the hydrogel according to the invention is characterized in that Tan δ is less than or equal to 0.05.

In an embodiment the hydrogel according to the invention is characterized in that Tan δ is less than or equal to 0.01.

In an embodiment the hydrogel according to the invention is characterized in that after swelling in water the cross-linked dextran polymer concentration is comprised from 0.01 to 0.2 g/g.

In an embodiment the hydrogel according to the invention is characterized in that after swelling in water the cross-linked dextran polymer concentration is comprised from 0.03 to 0.1 g/g.

In an embodiment the hydrogel according to the invention is characterized in that after swelling in water the cross-linked dextran polymer concentration is comprised from 0.05 to 0.1 g/g.

In an embodiment, the hydrogel is translucid.

In another embodiment the hydrogel is transparent.

In an embodiment, the hydrogel has a Young modulus comprised between 1 to 200 kPa.

In an embodiment, the hydrogel has a G' comprised from 0.5 to 70 kPa.

In an embodiment the hydrogel has a compression deformation at break of more than or equal to 10 %.

In an embodiment the hydrogel has a compression deformation at break of more than or equal to 15 %.

In an embodiment the hydrogel has a compression deformation at break of more than or equal to 20 %.

In an embodiment the hydrogel has a compression deformation at break of more than or equal to 25 %.

In an embodiment the hydrogel has a compression deformation at break of more than or equal to 30 %.

In an embodiment the hydrogel has a compression deformation at break of more than or equal to 35 %.

In an embodiment the hydrogel has a compression deformation at break of more than or equal to 40 %.

In an embodiment the hydrogel has a compression deformation at break of more than or equal to 45 %.

In an embodiment the hydrogel has a compression deformation at break of more than or equal to 50 %.

In an embodiment the hydrogel has a compression deformation at break of more than or equal to 55 %.

In an embodiment the hydrogel has a compression deformation at break of more than or equal to 60 %.

In an embodiment the hydrogel has a traction deformation at break of more than or equal to 10 %.

In an embodiment the hydrogel has a traction deformation at break of more than or equal to 15 %.

In an embodiment the hydrogel has a traction deformation at break of more than or equal to 20 %.

In an embodiment the hydrogel has a traction deformation at break of more than or equal to 25 %.

In an embodiment the hydrogel has a traction deformation at break of more than or equal to 30 %.

In an embodiment the hydrogel has a traction deformation at break of more than or equal to 35 %.

In an embodiment the hydrogel has a traction deformation at break of more than or equal to 40 %.

In an embodiment the hydrogel has a swelling ratio of more than 1.

In an embodiment the hydrogel has a swelling ratio of more than 1.1.

In an embodiment the hydrogel has a swelling ratio of more than or equal to 1.2.

In an embodiment the hydrogel has a swelling ratio of more than or equal to 1.3.

In an embodiment the hydrogel has a swelling ratio of more than or equal to 1.4.

In an embodiment the hydrogel has a swelling ratio of more than or equal to 1.5.

In an embodiment the hydrogel has a swelling ratio of more than or equal to 1.6.

In an embodiment the hydrogel has a swelling ratio of less than or equal to 5.

In an embodiment the hydrogel has a swelling ratio of less than or equal to 4.

In an embodiment the hydrogel has a swelling ratio of less than or equal to 3.

In an embodiment the hydrogel has a swelling ratio of less than or equal to 2.8.

In an embodiment the hydrogel has a water content of at least 80 wt%.

In an embodiment the hydrogel has a water content of at least 85 wt%.

In an embodiment the hydrogel has a water content of at least 90 wt%.

In an embodiment the hydrogel has a water content of at least 97 wt%.

In an embodiment the hydrogel has a water content of at least 96 wt%.

In an embodiment the hydrogel has a water content of at least 95 wt%.

In an embodiment the hydrogel has a water content of at least 94 wt%.

In an embodiment the hydrogel has a water content of at least 93 wt%.

In an embodiment the hydrogel has a water content of at most 99 wt%.

In an embodiment the hydrogel has a water content of at most 98 wt%.

In an embodiment the hydrogel according to the invention is characterized in that it further comprises biological cells.

In an embodiment the cells are cells from human or animal origin.

In an embodiment the cells are cell lines.

In an embodiment the cells are stem-cells derived.

In an embodiment the stem cells are chosen from embryonic-stem cells, from induced-pluripotent-stem-cells or from mesenchymal-stem-cells.

In an embodiment the cells are primary cells.

In an embodiment the cells are proteins, hormones or peptide secreting cells.

In an embodiment the cells are chosen from
- insulin secreting cells for diabetes treatment
- Factor VIII or Factor IX secreting cells for hemophilia treatment and
- β-glucocerebrosidase secreting cells for Gaucher disease.

In an embodiment the hydrogel according to the invention is characterized in that insulin secreting cells are chosen into the group of pancreatic cells.

In an embodiment the hydrogel according to the invention is characterized in that insulin secreting cells are Langherans islets.

In an embodiment the hydrogel according to the invention is characterized in that the biological cells are pseudo islets.

The invention also concerns the use of a cross-linked dextran copolymer according to the invention into the form of a hydrogel to prepare a cells composition.

In an embodiment the cells are chosen amongst one or multiple type of cells, either isolated or aggregated, which may secrete active principles.

The invention also concerns an implantable device comprising at least a hydrogel, characterized in that it further comprises biological cells.

he invention also concerns an implantable device comprising at least a hydrogel wherein the cells are chosen from
- insulin secreting cells for diabetes treatment
- Factor VIII or Factor IX secreting cells for hemophilia treatment and
- β-glucocerebrosidase secreting cells for Gaucher disease.

In an embodiment, the cross-linked dextran polymer according to the invention is a dextran polymer bearing carboxylate groups wherein the at least divalent radical L(-)ᵢ is covalently bound to the dextran polymer backbone with i -(R₁)ₘG₁- - radicals, wherein i is 2, 4 or 8.

In an embodiment, the cross-linked dextran polymer according to the invention is a dextran polymer bearing carboxylate groups wherein the at least divalent radical L(-)ᵢ is covalently bound to the dextran polymer backbone with i -(R₁)ₘG₁- - radicals, wherein, i is 2.

In an embodiment, the cross-linked dextran polymer according to the invention is a dextran polymer bearing carboxylate groups wherein the at least divalent radical L(-)ᵢ is covalently bound to the dextran polymer backbone with i -(R₁)ₘG₁- - radicals, wherein, i is 4.

The invention also concerns the precursor of the cross-linked dextran polymer which is a dextran polymer bearing carboxylate groups covalently bound to-(R₁)ₘG₂ monovalent radicals wherein -G₂ which is the precursor of radical -G₁- is a Michael acceptor.

In an embodiment, the precursor is a cross-linked dextran polymer as above defined wherein -G₂ is chosen amongst maleimide or vinylsulfone or a group comprising a maleimide or vinylsulfone.

In an embodiment, the precursor is a cross-linked dextran polymer as above defined wherein -G₂ is a maleimide.

The invention also concerns a process to synthetize a dextran bearing carboxylate groups and at least one -(R₁)ₘG₂ substituent
- wherein -G₂, a Michael acceptor, precursor of -G₁ is chosen amongst linear or branched or cyclic alkyl monovalent radical comprising from 1 to 6 carbon atoms and may comprise heteroatoms such as oxygen, nitrogen or sulfur,
- R₁ being defined as above,
comprising the steps of:
a) preparation of a solution of dextran bearing carboxylate groups,
b) substitution of the hydroxyl of the carboxylate group with -(R₁)ₘG₂.

In an embodiment, the dextran bearing carboxylate groups and at least one -(R₁)ₘG₂ substituent according to the invention is a dextran polymer wherein -G₂ is chosen amongst maleimide or vinylsulfone or a group comprising a maleimide or a vinylsulfone.

In particular embodiments the dextran bearing carboxylate groups and at least one -(R₁)ₘG₂ substituent is crosslinked with a precursor of the linker L(-)ᵢ.

The cross-linking step is performed by mixing the solution comprising the dextran bearing carboxylate groups and at least one -(R₁)ₘG₂ substituent and a solution of the precursor of the linker L(-)ᵢ

In an embodiment, the precursor of the linker L(-)ᵢ bears functional group ie -SH groups that react with a -(R₁)ₘG₂ group that comprises vinylsulfone or maleimide groups in a Michael addition reaction to give the cross-linked dextran polymer of the invention.

In an embodiment, the cross-linking step is performed with solutions that comprise from 1 to 150 mM, preferably from 10 to 100 mM of reactive functions - (R₁)ₘG₂ of the precursor of the dextran carboxylate.

In an embodiment, the cross-linking step is performed with solutions that comprise from 1 to 150 mM, preferably from 10 to 100 mM of reactive functions of the precursor of the linker L(-)ᵢ.

In an embodiment, in the cross-linking step, the solution of the dextran polymer precursor bearing reactive functions -(R₁)ₘG₂ and the solution of the linker L(-)ᵢ precursor are used in equimolar ratio (1:1).

In an embodiment the ratio between the reactive functions -(R₁)ₘG₂ and reactive functions of the precursor of L(-)ᵢ is comprised from 0.8 to 1.2.

In an embodiment the ratio between the reactive functions -(R₁)ₘG₂ and reactive functions of the precursor of L(-)ᵢ is comprised from 0.9 to 1.1.

In an embodiment the ratio between the reactive functions -(R₁)ₘG₂ and reactive functions of the precursor of L(-)ᵢ is 1.

The cross-linking step is a gelation step that leads to the formation of a hydrogel according to the invention.

The hydrogel formation kinetic is function of the temperature and could be modulated by the reactant's concentrations, pH and temperature.

In an embodiment the time to obtain a hydrogel according to the invention is comprised from 1 minute to 6 hours.

In an embodiment the cross-linking step is carried out for 1 hour.

In an embodiment the temperature of the cross-linking step is comprised from 4°C to room temperature and could vary between the step of mixing and the step of gelation and moulding.

In an embodiment the mixing is performed at 4°C and the gelation is carried out at room temperature for 1 hour.

In an embodiment, after cross-linking or gelation, the hydrogel is swelled in a buffer solution, the pH of the buffer solution is comprised from 5 to 8, preferably from 6 to 8 and more preferably from 6.8 to 7.5.

In an embodiment, the buffer solution is a PBS solution at pH 7.4.

In an embodiment, the buffer solution is a Tris solution at pH 8.

In an embodiment the swelling allows the hydrogel mass being increased by 1, 2, 3 or 4 compared to its initial mass.

The invention also concerns a process to synthetize a cross-linked dextran polymer according to the invention, into the form of a hydrogel, comprising the steps of:
a) preparation of a sterile solution comprising a dextran bearing methyl carboxylate groups according to formula I and at least two -(R₁)ₘG₂ radicals,
b) preparation of a sterile solution of mercaptopolyethyleneglycol or mercaptoethyl polyoxyethylene according to formula II, III, III', VII or VII'
c) addition of the sterile solution obtained from step b to the solution obtained from step a,
d) the addition being directly done in a mould or the solutions are introduced into a mould after being mixed,
e) cross-linking and gelation by Michael reaction, for example at room temperature or at 37°C,
f) unmoulding and swelling to obtain an hydrogel.

The invention also concerns a process to prepare the implantable device comprising at least a hydrogel according to the invention comprising the steps of:
g) directly do the addition step c in a mould or introduce the solutions into a mould after being mixed,
h) unmould the obtain hydrogel before swelling
i) obtain the implantable device comprising at least a hydrogel after the swelling step.

In an embodiment, the process to prepare the implantable device comprising at least a hydrogel according to the invention comprises the steps of cutting and shaping the swelled hydrogel obtain from step e or the implantable device comprising hydrogel form step h.

In an embodiment steps c) and d) are done simultaneously.

In an embodiment, the swelling is done into a PBS solution at pH 7,4.

In an embodiment of the process according to the invention an active pharmaceutical ingredient (API) is entrapped into the hydrogel.

The invention also concerns a therapeutic use of the implantable device comprising at least a hydrogel according to the invention as a therapeutic implant to administer the API to a mammal.

The invention also concerns a process to prepare the implantable device comprising at least a hydrogel that comprises biological cells comprising the steps of:
a) preparation of a sterile solution comprising a dextran bearing carboxylate groups according to formula I and at least two -(R₁)ₘG₂ radical,
b) preparation of a sterile solution of mercaptopolyethyleneglycol or mercaptoethyl polyoxyethylene according to formula II, III, III', VII or VII',
c) preparation of a suspension of biological cells,
d) mixing the biological cells suspension obtained from step c and the solution obtained from the step b or a,
e) addition of the sterile solution obtained from step a or b which is not used in step d to the solution obtained from step d,
f) the addition of step e being either done directly in a mould or the solutions are introduced into a mould after being mixed,
g) cross-linking and gelation by Michael reaction at room temperature,
h) unmoulding and swelling to obtain an hydrogel comprising biological cells.
i) obtaining the implantable device comprising at least a hydrogel that comprises biological cells after the swelling step.

In an embodiment, the swelling is done into a PBS solution at pH 7,4.

In an embodiment the implantable device comprising at least a hydrogel according to the invention is characterized in that it further comprises biological cells.

In an embodiment the cells are cells from human or animal origin.

In an embodiment the cells are cell lines.

In an embodiment the cells are stem-cells derived.

In an embodiment the stem cells are chosen from embryonic-stem cells, from induced-pluripotent-stem-cells or from mesenchymal-stem-cells.

In an embodiment the cells are primary cells.

In an embodiment the cells are protein(s), hormone(s) or peptide(s) secreting cells.

In an embodiment the cells are chosen from
- insulin secreting cells for diabetes treatment
- Factor VIII or Factor IX secreting cells for hemophilia treatment and
- β-glucocerebrosidase secreting cells for Gaucher disease.

In an embodiment the implantable device comprising at least a hydrogel according to the invention is characterized in that insulin secreting cells are chosen into the group of pancreatic cells.

In an embodiment the implantable device comprising at least a hydrogel according to the invention is characterized in that insulin secreting cells are Langherans islets.

In an embodiment the implantable device comprising at least a hydrogel according to the invention is characterized in that the biological cells are pseudo islets.

The invention also concerns a therapeutic use of the implantable device comprising at least a hydrogel according to the invention for treating a disorder or disease in a mammal wherein the disorder or disease is due to lack or malfunction of endocrine function of pancreas organ.

The invention also concerns an implantable device comprising at least a hydrogel for use as a medicament.

The invention also concerns an implantable device comprising at least a hydrogel for use in the treatment of a disease such as diabetes.

The invention also concerns a therapeutic method for treating a disorder or disease in a mammal wherein the disorder or disease is due to lack or malfunction of endocrine function of pancreas organ by implantation of the implantable device comprising at least a hydrogel according to the invention

The invention also concerns a therapeutic method for treating a disease such as diabetes by implantation of the implantable device comprising at least a hydrogel according to the invention

The invention also concerns an implantable device comprising at least a hydrogel according to the invention and obtained according to the process of the invention.

The invention also concerns an implant consisting of the implantable device comprising at least a hydrogel according to the invention.

In an embodiment, at least 50 % of the surface of the implantable device comprising at least a hydrogel is directly in contact with the medium in which it is implanted.

In an embodiment, at least 75 % of the surface of the implantable device comprising at least a hydrogel is directly in contact with the medium in which it is implanted.

In an embodiment, at least 90 % of the surface of the implantable device comprising at least a hydrogel is directly in contact with the medium in which it is implanted.

In an embodiment, at least 95 % of the surface of the implantable device comprising at least a hydrogell is directly in contact with the medium in which it is implanted.

In an embodiment, 99 % of the surface of the implantable device comprising at least a hydrogelis directly in contact with the medium in which it is implanted.

In an embodiment, at least 50 % of the surface of the implantable device comprising at least a hydrogel is directly in contact with the exterior of the device or implant.

By « directly in contact with the exterior » means there is no separation between the hydrogel and the exterior, for example no wall made of a non-hydrogel material between the hydrogel and the exterior of the implantable device or implant.

In an embodiment, at least 75 % of the surface the hydrogel is directly in contact with the exterior of the implantable device or implant.

In an embodiment, at least 90 % of the surface the hydrogel is directly in contact with the exterior of the implantable device or implant.

In an embodiment, at least 95 % of the surface the hydrogel is directly in contact with the exterior of the device or implant.

In an embodiment, at least 99 % of the surface the hydrogel is directly in contact with the exterior of the implantable device or implant.

In an embodiment, 100 % of the surface the hydrogel is directly in contact with the exterior of the implantable device or implant.

The cells or the API are entrapped into the maze of cross-linked dextran hydrogel.

In this specification the word "entrapped" is equivalent to "encapsulated" or "encapsulation".

The hydrogel matrix allows passage of small molecules e.g. nutrients and API, API being entrapped into the hydrogel or secreted by the entrapped cells.

Typically, API are hormone and peptide drugs chosen amongst PTH protein, insulin and coagulation factors.

In an embodiment, the mesh sizes of the matrix is immunoisolant and stops the T lymphocytes in order to preserve the cells.

In an embodiment, this mesh size is less than 1 µm.

In another embodiment it is less that 100 nanometers, preferably less than 10 nanometers, and more preferably around 5 nanometers.

### EXAMPLES

### Part A - CHEMISTRY

### Example A1 : Synthesis of substituted dextranmethylcarboxylate (DMCMal)

**Table 1. List of synthesized polysaccharides DMCMal**

| **Polysaccharides** | **Structure** |
|---|---|
| Polysaccharide DMCMal-1 | 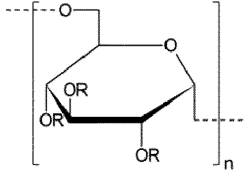 |
| | Mw (Dextran) = 40 kg/mol |
| | n = 205 |
| | 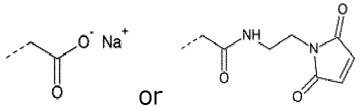 |
| | R = H or |
| | DS₂ = 1.2 |
| | DS₁ = 0.24 |
| Polysaccharide DMCMal-2 | 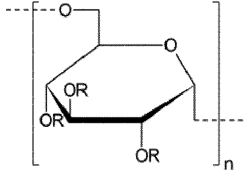 |
| | Mw (Dextran) = 40 kg/mol |
| | n = 205 |
| | 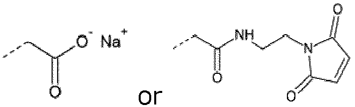 |
| | R = H or |
| | DS₂ = 2.25 |
| | DS₁ = 0.24 |
| Polysaccharide DMCMal-3 | 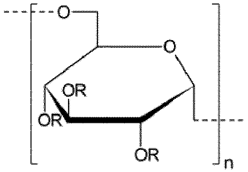 |
| | Mw (Dextran) = 500 kg/mol |
| | n = 2200 |
| | 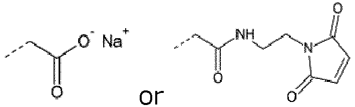 |
| | R = H or |
| | DS₂ = 1.0 |
| | DS₁ = 0.25 |
| Polysaccharide DMCMal-4 | 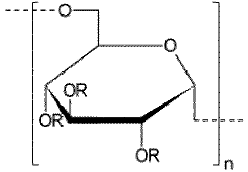 |
| | Mw (Dextran) = 500 kg/mol |
| | n = 2200 |
| | 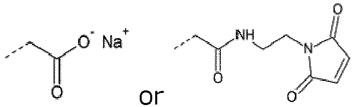 |
| | R = H or |
| | DS₂ = 1.0 |
| | DS₁ = 0.09 |

### Polysaccharide DMCMal-1

### Polysaccharide DMC-1:

65 g (0.4 mol of glucoside units, 1.2 mol of hydroxyl functional groups) of dextran having a weight-average molar mass of 40 kg/mol (Pharmacosmos, degree of polymerization n= 205), are dissolved in water (285 g/L) at 30 °C, then NaBH₄ (74 mg, 1.95 mmol) is added and the mixture is stirred at 30 °C for 2 h. To this solution is added sodium chloroacetate (140 g, 1.2 mol) and the mixture is heated at 65 °C for 1 h. 10 N NaOH (200 mL, 2 mol) is then slowly added over 1.5 h and the mixture is stirred at 65 °C for 1 h. The mixture is diluted with water (120 mL), cooled to room temperature, neutralized with acetic acid and then purified by ultrafiltration on PES membrane (MWCO 5 kDa) against phosphate buffer pH 7, then water. The polysaccharide DMC-1 concentration of the final solution is determined by dry extract, and then an acid/base assay is carried out in order to determine the degree of substitution with methyl carboxylate.

According to the dry extract: [polysaccharide DMC-1] = 50.6 mg/g

According to the acid/base assay, degree of substitution with methylcarboxylate (DS₂) = 1.2

### Polysaccharide DMCMal-1:

To 158 g of the solution of polysaccharide DMC-1 obtained above (50.6 mg/g, DS₂ = 1.2, 10.0 g of polysaccharide DMC-1, 38.73 mmol of glucoside units) 2-hydroxypyridine 1-oxide (HOPO) (4.30 g, 38.73 mmol) is added and the mixture is cooled to 4 °C. To this solution are added *N*-(2-aminoethyl)maleimide hydrochloride (Mal) (2.05 g, 11.62 mmol), triethylamine (Et₃N) (1.62 mL, 11.62 mmol) and N-ethyl-*N*'-(3-dimethylaminopropyl)carbodiimide hydrochloride (EDC) (7.42 g, 38.73 mmol) and the reaction mixture is stirred at 4 °C for 4 h. Two additional additions of EDC (7.42 g, 38.73 mmol) are processed every 4 h. The mixture is diluted with phosphate buffer pH 7, then purified by ultrafiltration on PES membrane (MWCO 5 kDa) against phosphate buffer pH 7, NaCl (9 g/L) in water and then water. The polysaccharide DMCMal-1 concentration of the final solution is determined by dry extract, and the degree of substitution with maleimide is determined by ¹H NMR in D₂O. The final solution is stored at -20 °C or freezed-dried.

According to the dry extract: [polysaccharide DMCMal-1] = 18.0 mg/g

According to ¹H NMR (D₂O), degree of substitution with maleimide (DS₁) = 0.24

### Polysaccharide DMCMal-2

### Polysaccharide DMC-2:

90 g (0.35 mol of glucoside units) of freeze-dried polysaccharide DMC-1 are dissolved in water (260 g/L) at 65 °C, then sodium chloroacetate (204 g, 1.75 mol) is added and the mixture is maintained at 65 °C for 1 h. 10 N NaOH (175 mL, 1.75 mol) is then slowly added over 1 h and the mixture is stirred at 65 °C for a further hour. Another portion of sodium chloroacetate (122 g, 1.05 mol) is then added and the mixture is maintained at 65 °C for 0.5 h. 10 N NaOH (105 mL, 1.05 mol) is then slowly added over 1 h and the mixture is stirred at 65 °C for a further 1 h. The mixture is diluted with water, cooled to room temperature, neutralized with acetic acid and then purified by ultrafiltration on PES membrane (MWCO 5 kDa) against phosphate buffer pH 7, then water. The polysaccharide DMC-2 concentration of the final solution is determined by dry extract, and then an acid/base assay is carried out in order to determine the degree of substitution with methyl carboxylate.

According to the dry extract: [polysaccharide DMC-2] = 48.8 mg/g

According to the acid/base assay, degree of substitution with methylcarboxylate (DS₂) = 2.25

### Polysaccharide DMCMal-2:

Using a process similar to the one used for the preparation of polysaccharide DMCMal-1, starting from polysaccharide DMC-2 (48.8 mg/g, DS₂ = 2.25, 10.0 g, 29.22 mmol of glucoside units) and with *N*-(2-aminoethyl)maleimide hydrochloride (1.55 g, 8.77 mmol), polysaccharide DMCMal-2 is obtained.

According to the dry extract: [polysaccharide DMC-2] = 17.9 mg/g

According to ¹H NMR (D₂O), degree of substitution with maleimide (DS₁) = 0.24

### Polysaccharide DMCMal-3

### Polysaccharide DMC-3:

Using a process similar to the one used for the preparation of polysaccharide DMC-1, starting from a dextran having a weight-average molar mass of 500 kg/mol, polysaccharide DMC-3 is obtained.

According to the dry extract: [polysaccharide DMC-3] = 34.3 mg/g

According to the acid/base assay, degree of substitution with methylcarboxylate (DS₂) = 1.0

### Polysaccharide DMCMal-3:

Using a process similar to the one used for the preparation of polysaccharide DMCMal-1, starting from polysaccharide DMC-3 (34.3 mg/g, DS₂ = 1.0, 10.0 g, 41.29 mmol of glucoside units) and with *N*-(2-aminoethyl)maleimide hydrochloride (2.19 g, 12.39 mmol), polysaccharide DMCMal-3 is obtained.

According to the dry extract: [polysaccharide DMCMal-3] = 12.0 mg/g

According to ¹H NMR (D₂O), degree of substitution with maleimide (DS₁) = 0.25

### Polysaccharide DMCMal-4

Using a process similar to the one used for the preparation of polysaccharide DMCMal-1, starting from polysaccharide DMC-3 (34.3 mg/g, DS₂ = 1.0, 10.0 g, 41.29 mmol of glucoside units) and with *N*-(2-aminoethyl)maleimide hydrochloride (729 mg, 4.13 mmol), polysaccharide DMCMal-4 is obtained.

According to the dry extract: [polysaccharide DMCMal-4] = 18.0 mg/g

According to ¹H NMR (D₂O), degree of substitution with maleimide (DS₁) = 0.09

### Example A2 : Polyethylene glycol derivatives comprising at least two thiol functions (called in this specification "PEG-SH")

Commercial PEG derivatives functionalized with thiol (SH) groups were purchased. Linear homo bifunctional PEG-SH and multi-arm homofunctional PEG-SH having different molecular weights were used and are shown in the following Table 2.

**Table 2 : List of commercial PEG-SH used**

| PEG-SH | Chemical Name | Thiol groups | Mn (kg/mol) |
|---|---|---|---|
| PEG-SH-1 | Poly(ethylene glycol) dithiol | 2 | 3.4 |
| PEG-SH-2 | Poly(ethylene glycol) dithiol | 2 | 1 |
| PEG-SH-3 | Pentaerythritol tetra(mercaptoethyl) polyoxyethylene | 4 | 5.2 |
| PEG-SH-4 | Pentaerythritol tetra(mercaptoethyl) polyoxyethylene | 4 | 20 |

### Part B - BIOLOGY

### Example B1: Preparation of Beta-TC-tet Cell pseudoislets

Beta-TC-tet cell line (ATCC) was cultivated in culture medium (DMEM supplemented with 15% Horse serum, 2.5% Fetal Bovine Serum, and 1% Penicilin/Streptomycin) in an incubator at 37 °C and 5% CO₂. Cells were subcultured 2-3 times a week using 0.05% trypsin / EDTA to detach the cells and diluted 5 times in culture medium.

Pseudoislets of 150 µm were formed with Beta-TC-tet cell line using micropatterned plastic plates with 500 µm-wide and 400 µm-deep microwells (Eplasia - Corning) by seeding 400 cells per microwell and incubating them at 37 °C and 5% CO₂ for 3 days. Pseudoislets were then collected, concentrated by centrifugation, and finally resuspended in 0.9% NaCl.

### Example B2: Preparation of Hep-G2 Cell pseudoislets

Hep-G2 cell line (ATCC) was cultivated in culture medium (EMEM supplemented with 10% Fetal Bovine Serum and 1% Penicilin/Streptomycin) in an incubator at 37 °C and 5% CO₂. Cells were subcultured 2-3 times a week using 0.05% trypsin / EDTA to detach the cells and diluted 5 times in culture medium.

Pseudoilets of 150 µm were formed with Hep-G2 cell line using micropatterned plastic plates with 500 µm wide and 400 µm deep microwells (Eplasia - Corning) by seeding 400 cells per microwell and incubating them at 37 °C and 5% CO₂ for 3 days. Pseudoislets were then collected, concentrated by centrifugation, and finally resuspended in 0.9% NaCl.

### Example B3: Preparation of isolated primary human islets

Pancreata were procured in human brain-dead donors.

Pancreatic islets were procured following the method described in Technique of pancreatic procurement for pancreatic islet isolation, Pattou et al., Anchir 2005. Briefly, the pancreas was isolated from the tissue, and perfused in the Virsung canal with collagenase. The islets are then purified through density grandient centrifugation. Purified islets are then cultured in culture flasks in CMRL medium (Gibco) supplemented with 0.1% BSA at 37°C under 5% CO₂.

### Part C - PHYSICO-CHEMISTRY

### Example C1: Preparation of solutions of concentrated polysaccharide DMCMal

A concentrated polysaccharide solution is prepared by weighing the appropriate weight of a sterile freeze-dried polysaccharide obtained according to part A1 and adding the appropriate weight of sterile deionised water. The solution is placed on an orbital shaker overnight at 70 rpm for complete solubilization. The pH of the solution is adjusted to pH 3, pH 4 or pH 5 by addition of concentrated HCl (4 mol/L). The mass concentration of the solution of polysaccharide DMCMal (mg/g) is determined by dry exact. The volume concentration of the solution of polysaccharide DMCMal (mg/mL) is determined by density measurements, weighing three times 100 µL of solution. The solution is frozen at -20 °C until being used.

### Example C2: Preparation of solutions of concentrated PEG-SH

A concentrated solution of PEG-SH (from the list according to table 2) is prepared by weighing the appropriate weight of a PEG-SH powder and adding the appropriate weight of sterile deionised water. The solution is placed on roller shaker at 15 rpm for 2 h for complete solubilization before sterile filtration (0.22 µm). The mass concentration of the PEG-SH solution (mg/g) is determined by dry exact. The volume concentration of the PEG-SH solution (mg/mL) is determined by density measurements, weighing three times 100 µL of solution. The solution is frozen at -20 °C until being used.

### Example C3: Preparation of an implantable device comprising at least a hydrogel with microstructures

Hydrogel preparation is made in an aseptic environment.

Polysaccharide DMCMal and PEG-SH concentrated sterile solutions described in example C1 and example C2, respectively, are diluted with concentrated solution of NaCl and water to obtain isotonic solutions containing the desired concentration of polymer. The solutions are then equilibrated at 4 °C.
150 µL of isotonic concentrated solution of PEG-SH is added to 150 µL of isotonic concentrated solution of polysaccharide DMCMal in a 2 mL Eppendorf.The solutions are mixed with a pipette and 270 µL of the mixture are deposited on a glass slide within in a square silicone isolator (24x24 mm and 0.5 mm thick) sticked on the glass and delimiting the gel area.

**Table 3 : Hydrogel prepared according to example C3**

| Example | Polysaccharide | PEG(Thiol) / Mn | Mal: Thiol (mM:mM) |
|---|---|---|---|
| C3-1 | DMCMal-2 | PEG-SH-4 | 18:18 |

A 24×24mm PDMS square stamp bearing circular pillar structures with a diameter of 500 µm, edge to edge spacing of 500 µm and height of 500 µm is pressed against the solution within the square silicone isolator area for contact printing. After 15 min gelation at 20-25 °C the stamp is removed and the micro-structured hydrogel is unmoulded and introduced in PBS (phosphate buffer saline) overnight at 37 °C for swelling. The hydrogel piece is stored in 10 mL PBS at 4 °C until use.

Optionally the PBS solution contain 10 mM of cysteine. The hydrogel is rinsed with a 10 mL of PBS solution without cysteine and further immersed in 20 mL of the PBS solution overnight at 37 °C. The hydrogel piece is then stored in 10 mL of PBS solution at 4 °C until being used.

The microstructured device comprising a hydrogel is a flexible 500 µm thick hydrogel sheet containing holes close to 500 µm in diameter separated by distances close to 500 µm is obtained. By flexible is meant that the sheet can be gently rolled up without breaking.

### Example C4: Incorporation of Cell spheroids in micro-structured devices

Microstructured devices incorporating pseudoisletswere prepared in a similar process than in example C3, using the pseudoislets preparation method described in example B1 or example B2.

Polysaccharide DMCMal and PEG-SH concentrated sterile solutions described in example C1 and example C2, respectively, are diluted with a concentrated solution of NaCl and water to obtain isotonic solutions containing desired concentration of each DMC-Mal et PEG-SH. 75 µL of concentrated isotonic solution of PEG-SH is added to 75 µL of a concentrated suspension of spheroids from example B1 or B2. 150 µL of the suspension of pseudoislets in PEG-SH solution is added to 150 µL of isotonic concentrated solution of polysaccharide DMC-Mal in a 2 mL Eppendorf.

The solutions are mixed with a pipette and 270 µL of the mixture is deposited on a glass slide within in a square silicone isolator (24×24 mm and 0.5 mm thick) sticked on the glass and delimiting the gel area.

**Table 4: Hydrogels incorporating cell spheroids**

| Example | Polysaccharide | PEG(Thiol) / Mn | Mal: Thiol (mM:mM) | Cells |
|---|---|---|---|---|
| C4-1 | DMCMal-2 | PEG-SH-4 | 18:18 | Hep-G2 |
| C4-2 | DMCMal-2 | PEG-SH-4 | 18:18 | Beta-TC-tet |

A 24×24 mm PDMS square stamp bearing circular pillar structures with a diameter of 500 µm, edge to edge spacing of 500 µm and height of 500 µm is pressed against the solution within the square silicone isolator area for contact printing. After 15 min gelation at 20-25 °C the stamp is removed. Obervation of the hydrogel with a binocular magnifier confirms that pseudoisletsare incorporated within the micro-structured hydrogels.

The micro-structured device incorporating pseudoisletsis then immersed in 10 mL PBS before biological evaluation.

### Example C5: Viability of Cell spheroids incorporated in micro-structured device

The encapsulated pseudoisletswere stained with a Live/Dead staining (ThermoFisher) by supplementing the culture medium with 1 µM calcein-AM and 8 µM ethidium bromide for 30 min. After a washing step, pseudoislets were then imaged using epifluorescence microscopy with a 5X magnification.

The cytotoxicity ratio of the red area (dead cells) over the green area (living cells) was calculated. The viability was calculated by calculating 1 - cytotoxicity ratio.

**Table 5 : Cell viability of pseudoisletsincorporated in micro-structured hydrogels**

| Example | Hydrogels | Viability(% of living cells) | Cell viability (CV) |
|---|---|---|---|
| C5-1 | C4-1 | 84% | 23% |
| C5-2 | C4-1 | 92% | 5% |

The cell viability is excellent.

### Example C6: Incorporation of human pancreatic islets in micro-structured devices

Microstructured devices incorporating primary human islets were prepared in a similar process than in example C3, using the primary human islets preparation method described in example B3.

Polysaccharide DMCMal and PEG-SH concentrated sterile solutions described in example C1 and example C2, respectively, are diluted with a concentrated solution of NaCl and water to obtain isotonic solutions containing desired concentration of each DMC-Mal et PEG-SH. 56 µL of concentrated isotonic solution of PEG-SH is added to 56 µL of a concentrated suspension of human primary islets from example B3. 112 µL of the suspension of pseudoislets in PEG-SH solution is added to 48 µL of isotonic concentrated solution of polysaccharide DMC-Mal in a 2 mL Eppendorf.

The solutions are mixed with a pipette and 140 µL of the mixture is deposited on a glass slide within in a disk-shaped silicone isolator (2 cm diameter and 0.5 mm thick) sticked on the glass and delimiting the gel area.

A 2 cm disk-shaped PDMS stamp bearing circular pillar structures with a diameter of 500 µm, edge to edge spacing of 500 µm and height of 500 µm is pressed against the solution within the circular silicone isolator area for contact printing. After 30 min gelation at 20-25 °C the stamp is removed. The micro-structured hydrogel is unmoulded and introduced in an isotonic buffer at pH 8 containing Tris 150 mM/NaCl 30 mM /Cystein 10 mM at 20-25°C. After 15 minutes the tris/NaCl/Cystein buffer is replaced by cell culture media and the the hydrogels incorporating primary human islets are stored at 37°C until implantation.

The microstructured device is a foldable 500 µm thick hydrogel disc containing holes close to 500 µm in diameter separated by distances close to 500 µm.

### Example C7: Implantation of microstructured device

Disk-shaped microstructured devices as prepared in example C6, having 20 mm diameter and 500 µm thickness e, have been implanted in rats extra peritoneally in contact with the muscles. This was performed by mediane laparotomy, the parietal peritoneum being incised laterally allowing a pocket to be created between the peritoneum and the muscularis aponeurosis.The micro-structured hydrogels were placed inside this surgically created pocket, which was then closed using 2 to 4 non absorbable stitches.

After 28 days, the abdominal cavity was free of any adhesions, no inflammatory reaction was observed neither on the peritoneum, nor on the abdominal organs surface and a newly formed tissue was surrounding the device.

The histopathological analysis of the explanted devices concluded that almost no fibrotic reaction between the muscles and the implants was observed.

However, a peripheral reaction surrounded the implant was observed. This reaction was very limited as the a newly formed tissue which was observed:
- was very thin (thickness e of around 50 to 75 µm) and
- had non inflammatory cellular characteristics, ie no inflammatory cells had been detected.

This newly formed tissue surrounded all the surface of the hydrogel and thus did fill the channels of the hydrogel, creating a kind of "honeycomb-like structure". As all the surface of the hydrogel is surrounded by this newly formed collagenic tissue and as the design of the hydrogel allows a diminution of the mean distance between the surface of the hydrogel and the encapsulated cells, the vascularisation and nutrition of the encapsulated cells can be improved.

## Claims

1. Implantable device comprising at least a hydrogel comprising:
- an upper face,
- a bottom face parallel to the upper face, the distance between the upper and bottom faces defining a thickness (e) of the device
- at least one lateral face between the upper and bottom faces, and
- an array of channels within the hydrogel, each channel extending from the bottom face and/or from the upper face,
**characterized in that**
- the distance (d) between two neighboring channels of the array of channels, according to a dimension orthogonal to the thickness (e) of hydrogel, is inferior or equal to 1000 µm.

2. Implantable device comprising at least a hydrogel according to claim 1, wherein the distance between the at least one lateral wall and at least one channel of the array of channels is inferior or equal to 500 µm.

3. Implantable device comprising at least a hydrogel according to claim 2, wherein the array of channels includes:
- central channels
- peripheral channels which are closer to the at least one lateral face than the central channels,
- the distance (d") between each peripheral channel and the at least one lateral face being inferior or equal to 500µm.

4. Implantable device comprising at least a hydrogel according to anyone of claims 1 to 3, wherein at least one channel of the array of channels consists in a through hole extending between the upper and bottom faces.

5. Implantable device comprising at least a hydrogel according to anyone of claims 1 to 4, wherein at least one channel of the array of channels consists in a blind hole extending from either the upper face or the bottom face.

6. Implantable device comprising at least a hydrogel according to the invention, **characterized in that** at least 80 %, 85%, 90%, 95%, 98%, 99%, 100% of its volume is at most at 1 µm of the closest surface / interface between the hydrogel and the outside of the hydrogel.

7. Implantable device comprising at least a hydrogel according to any one of Claims 1 to 6, **characterized in that** the depth (H") of the channels is at least 50%, 60%, 70%, 80%, 90% or even 100% of the thickness (e) of the hydrogel.

8. Implantable device comprising at least a hydrogel according to any one of Claims 1 to 7, **characterized in that** the height of the channel is at least 40 %, 50 %, 60 %, 70%, 80 %, 90 % or even 95 % of the thickness (e) of the hydrogel.

9. Implantable device comprising at least a hydrogel according to any one of Claims 1 to 8, **characterized in that** the channels are cavities or grooves (g).

10. Implantable device comprising at least a hydrogel according to Claim 4 or 5, **characterized in that** the holes (p) have a diameter (∅) comprised between 50 to 1000 µm, 100 to 500 µm, 250 to 500 µm.

11. Implantable device comprising at least a hydrogel according to any one of the preceding claims, wherein the hydrogel is a crosslinked dextran polymer, bearing carboxylate groups, wherein at least two saccharidic units of dextran belonging to two different polymer chains are covalently linked by at least one at least divalent radical L(-)ᵢ, this at least divalent radical being a linear, branched or cyclic alkyl radical comprising at least 15 carbon atoms and optionally heteroatoms such as oxygen, nitrogen or sulfur.

12. Implantable device comprising at least a hydrogel accrording to claim 11, wherein the at least divalent radical -L(-)ᵢ is covalently bound to the dextran polymer backbone with -(R₁)ₘG₁- - radicals, wherein,
- L(-)ᵢ is a linear or branched polyether bearing at its ends, heteroatoms such as oxygen, nitrogen or sulfur,
- i is the valence of L and the number of -(R₁)ₘG₁- - radicals and is an integer comprised from 2 to 8 (2 ≤ i ≤ 8)
- m is an integer equal to 0 or 1,
- -R₁- is a linear or branched alkyl divalent radical comprising from 1 to 6 carbon atoms and optionally heteroatoms such as oxygen, nitrogen or sulfur.
- -G₁- is a linear or branched or cyclic alkyl divalent radical comprising from 1 to 6 carbon atoms and may comprise heteroatoms such as oxygen, nitrogen or sulfur.

13. Implantable device comprising at least a hydrogel according to any one of the preceding claims, **characterized in that** it further comprises biological cells.

14. Implantable device comprising at least a hydrogel accrording to claim 13, wherein the cells are chosen from
- insulin secreting cells for diabetes treatment
- Factor VIII or Factor IX secreting cells for hemophilia treatment and
- β-glucocerebrosidase secreting cells for Gaucher disease.

15. Implantable device comprising at least a hydrogel according to to any one of the preceding claims, for use in the treatment of a disorder or disease in a mammal wherein the disorder or disease is due to lack or malfunction of endocrine function of pancreas organ.

16. Implantable device comprising at least a hydrogel according to to any one of the preceding claims, for use in the treatment of a disease such as diabetes
